# FASCICULE DE BREVET EUROPEEN

(11) **EP 0 998 568 B1**
(45) Date de publication et mention de la délivrance du brevet: **07.12.2005**
(21) Numéro de dépôt: 99914603.8
(22) Date de dépôt: 16.04.1999
(51) Int. Cl.: C12N 15/54, C12N 9/10, C12N 15/62, C12N 15/86, C12N 7/01, C12N 5/10, A61K 48/00, A61K 38/45

(54) **MUTANT AYANT UNE ACTIVITE URACILE PHOSPHORIBOSYL TRANSFERASE**
URACYL-PHOSPHORIBOSYLTRANSFERASE MUTANTE
MUTANT HAVING URACIL PHOSPHORIBOSYL TRANSFERASE ACTIVITY

(30) Priorité: 17.04.1998 FR 9805054
(43) Date de publication de la demande: 10.05.2000
(73) Titulaire: TRANSGENE S.A., 67000 Strasbourg (FR)
(72) Inventeur: ERBS, Philippe, F-67000 Strasbourg (FR); JUND, Richard, F-67100 Strasbourg (FR)
(74) Mandataire: Guerre, Dominique
(86) Numéro de dépôt international: PCT/FR1999/000904
(87) Numéro de publication internationale: WO 1999/054481

(56) Documents cités:
- WO-A-96/16183
- KERN L ET AL: "The FUR1 gene of Saccharomyces cerevisiae: cloning, structure and expression of wild-type and mutant alleles." GENE, (1990 APR 16) 88 (2) 149-57. JOURNAL CODE: FOP. ISSN: 0378-1119., XP002087000 Netherlands

## Description

La présente invention a pour objet un polypeptide ayant une activité uracile phosphoribosyl transférase (UPRTase) dérivant d'une UPRTase native par mutation d'un ou plusieurs résidus de ladite UPRTase. Elle concerne également une séquence nucléotidique codant pour ce mutant UPRTase, un vecteur pour l'expression de cette dernière, une particule virale et une cellule hôte ainsi queune composition les comprenant. Enfin, elle vise également leur utilisation thérapeutique ainsi qu'une méthode de traitement les mettant en oeuvre. La présente invention est particulièrement utile dans le cadre de la thérapie par gènes suicides pour une application à l'égard notamment des maladies prolifératives et infectieuses.

La thérapie génique se définit comme le transfert d'information génétique dans une cellule ou un organisme hôte. Le premier protocole appliqué à l'homme a été initié aux Etats-Unis en septembre 1990 sur un patient génétiquement immunodéficient en raison d'une mutation affectant le gène codant pour l'Adénine Désaminase (ADA). Le succès relatif de cette première expérimentation a encouragé le développement de cette approche pour diverses maladies aussi bien génétiques (dans le but de corriger le dysfonctionnement d'un gène défectueux) qu'acquises (cancers, maladies infectieuses comme le SIDA...). Cette technologie a connu depuis lors de nombreux développements et parmi ceux-ci, la thérapie par « gènes suicides » qui utilise des gènes dont les produits d'expression sont capables de transformer une substance inactive (prédrogue) en substance cytotoxique, provoquant ainsi la mort cellulaire. En 1992, plusieurs équipes ont démontré l'intérêt de cette nouvelle approche pour traiter des tumeurs et inhiber la dissémination du virus HIV responsable du SIDA.

A cet égard, le gène codant pour la thymidine kinase du virus herpès simplex de type 1 (TK HSV-1) constitue le prototype des gènes suicides (Caruso et al., 1993, Proc. Natl. Acad. Sci. USA *90*, 7024-7028 ; Culver et al., 1992, Science *256*, 1550-1552 ; Ram et al., 1997, Nat. Med. *3*, 1354-1361). Le polypeptide TK n'est pas toxique en tant que tel mais il catalyse la transformation d'analogues de nucléosides tels que l'acyclovir ou le ganciclovir (GCV). Les nucléosides modifiés sont incorporés dans les chaînes d'ADN en élongation avec pour conséquence l'inhibition de la division cellulaire. De nombreux couples gène suicide / prédrogue sont actuellement disponibles. On peut citer plus particulièrement, le cytochrome p450 de rat et la cyclophosphophamide (Wei et al., 1994, Human Gene Therapy 5, 969-978), la purine nucleoside phosphorylase d'*Escherichia coli (E. Coli)* et la 6-methylpurine deoxyribonucleoside (Sorscher et al., 1994, Gene Therapy *1*, 233-238), la guanine phosphoribosyl transférase d'*E*. *coli* et la 6- thioxanthine (Mzoz et Moolten, 1993, Human Gene Therapy *4*, 589-595) et la cytosine désaminase (CDase) et la 5-fluorocytosine (5FC).

La CDase intervient dans la voie métabolique des pyrimidines par laquelle la cytosine exogène est transformée par le biais d'une désamination hydrolytique en uracile. Des activités CDases ont été mises en évidence chez les procaryotes et les eucaryotes inférieurs (Jund et Lacroute, 1970, J. Bacteriol. 102, 607-615 ; Beck et al., 1972, J. Bacteriol. *110*, 219-228 ; De Haan et al., 1972, Antonie van Leeuwenhoek *38*, 257-263 ; Hoeprich et al., 1974, J. Inf. Dis. 130, 112-118 ; Esders et Lynn, 1985, J. Biol. Chem. *260*, 3915-3922) mais sont absentes chez les mammifères (Koechlin et al., 1966, Biochem Pharmacol. *15*, 435-446 ; Polak et al., 1976, Chemotherapy 22, 137-153). Les gènes *FCY1* de *Saccharomyces cerevisiae (S. cerevisiae*) et *codA d'E. coli* codant respectivement pour la CDase de ces deux organismes sont connus et leurs séquences publiées (EP 402 108 ; Erbs et al., 1997, Curr. Genet. *31*, 1-6 ; WO93/01281).

La CDase désamine également un analogue de la cytosine, la 5-fluorocytosine (5-FC) en 5-fluorouracile (5-FU) composé hautement cytotoxique quand il est converti en 5-fluoro-UMP (5-FUMP). Les cellules dépourvues d'activité CDase, en raison soit d'une mutation inactivante du gène codant pour l'enzyme, soit de leur déficience naturelle pour cette enzyme comme le sont les cellules mammifères, sont résistantes au 5-FC (Jund et Lacroute, 1970, J. Bacteriol. *102*, 607-615 ; Kilstrup et al., 1989, J. Bacteriol. 1989 *171*, 2124-2127). Par contre, les cellules mammifères dans lesquelles ont été transférées les séquences codant pour une activité CDase ont acquis une sensibilité au 5-FC (Huber et al., 1993, Cancer Res. *53*, 4619-4626 ; Mullen et al., 1992, Proc. Natl. Acad. Sci. USA *89*, 33-37 ; WO 93/01281). De plus, les cellules avoisinantes non transformées deviennent également sensibles au 5-FC (Huber et al., 1994, Proc. Natl. Acad. Sci. USA *91*, 8302-8306). Ce phénomène, appelé effet de voisinage (bystander en anglais), est dû à l'excrétion par les cellules exprimant l'activité CDase, de 5-FU qui intoxique les cellules voisines par simple diffusion à travers la membrane plasmique. Cette propriété de diffusion passive du 5-FU constitue un avantage par rapport au système de référence *tk*/GCV pour lequel l'effet de voisinage nécessite un contact avec les cellules qui expriment tk (Mesnil et al., 1996. Proc. Natl. Acad. Sci. USA 93, 1831-1835). On conçoit donc tous les atouts que présentent la CDase dans le cadre de la thérapie génique, notamment anticancéreuse.

Cependant, la sensibilité au 5-FC varie beaucoup selon les lignées cellulaires. Une faible sensibilité est observée par exemple dans des lignées tumorales humaines PANC-1 (carcinome de pancréas) et SK-BR-3 (adénocarcinome du sein) transduites par un rétrovirus exprimant le gène *codA d'E. Coli* (Harris et al., 1994, Gene Therapy *1*, 170-175). Ce phénomène indésirable pourrait s'expliquer par l'absence ou la faible conversion endogène du 5-FU formé par l'action enzymatique de la CDase en 5-FUMP cytotoxique. Cette étape, normalement assurée dans les cellules mammifères par l'orotate phosphorybosyl transférase (Peters et al., 1991, Cancer 68, 1903-1909), peut être absente dans certaines tumeurs et rendre ainsi la thérapie génique, basée sur la CDase, inopérante.

Chez les procaryotes et eucaryotes inférieurs, l'uracile est transformée en UMP par l'action de l'uracile phosphoribosyl transférase (UPRTase). Cette enzyme convertit également le 5-FU en 5-FUMP. Ainsi des mutants *fur1* de la levure *S. cerevisiae* sont résistants à de fortes concentrations de 5-FU (10 mM) et de 5-FC (10 mM) car en absence d'activité UPRTase, le 5-FU, provenant de la désamination du 5-FC par la CDase, n'est pas transformé en 5-FUMP cytotoxique (Jund et Lacroute, 1970, J. Bacteriol. *102*, 607-615). Les gènes *upp* et *FUR1* codant pour l'UPRTase respectivement d'*E. coli* et de *S. cerevisiae* ont été clonés et séquencés (Andersen et al., 1992, Eur. J. Biochem. *204*, 51-56 ; Kern et al., 1990, Gene *88*, 149-157).

Pour remédier à ces inconvénients. le document de l'art antérieur WO-A-96/16183 préconise l'utilisation d'une protéine de fusion codant pour une enzyme à deux domaines ayant les activités CDase et UPRTase et démontre *in vitro* que le transfert d'un gène hybride *codA::upp* ou *FCY1::FUR1* porté par un plasmide d'expression augmente la sensibilisation au 5-FC de cellules B16 transfectées.

La présente invention est une amélioration de la technique antérieure en ce qu'elle utilise un gène *FUR1* muté codant pour une UPRTase délétée dans sa partie N-terminale. La présente invention résulte de l'observation selon laquelle le gène *FUR1* contient après le codon ATG initiateur, un deuxième codon ATG codant pour la méthionine en position 36 de la protéine native.

On a maintenant construit un gène *FUR1* dépourvu de 105 nucléotides en 5' de la partie codante permettant la synthèse d'une UPRTase délétée des 35 premiers résidus en position N-terminale et débutant à la méthionine en position 36 dans la protéine native. On a montré que le produit d'expression du gène mutant, désigné *FUR1Δ105*, peut complémenter un mutant *fur1* de *S. cerevisiae*, ce qui démontre sa fonctionnalité. De manière surprenante, le mutant tronqué présente une activité UPRTase supérieure à celle de l'enzyme native, comme en témoignent les dosages enzymatiques réalisés sur des cellules COS7 transfectées par un plasmide d'expression du gène correspondant (gène *FUR1Δ105* comparé au sauvage). Trois lignées tumorales humaines, choisies à cause de leur résistance au 5-FU ont été transduites par le gène mutant transporté par un vecteur adénoviral et présentent *in vitro* une sensibilité accrue au 5-FU. La sensibilité au 5-FC est également augmentée si les cellules sont coinfectées par des adénovirus exprimant respectivement les gènes *FCY1* et *FUR1Δ105*par rapport à une infection par un adénovirus exprimant *FCY1* seul. De manière encore plus surprenante, la protéine de fusion produite par le gène hybride *FCY1::FUR1Δ105* résultant de la fusion en phase des gènes *FCY1* et *FUR1* tronqué possède une activité UPRTase conservée mais présentant une activité CDase augmentée d'un facteur 10 à 30 par rapport à celle mesurée avec le produit *FCY1* natif. La forte activité CDase de la protéine bifonctionnelle permet la formation d'un pool de 5-FU générant un effet « bystander » important. Il est à noter qu'aucune amélioration de l'activité CDase n'a été démontrée pour la protéine de fusion CDase::UPRTase de WO-A-96/16183.

La présente invention fournit un mutant plus efficace permettant d'augmenter la sensibilité des cellules à la 5-FC et d'améliorer les perspectives de thérapie génique par gènes suicides. Ce mutant est utile pour de nombreuses applications, notamment anticancéreuses, antivirales et toutes applications nécessitant la mort cellulaire.

C'est pourquoi la présente invention a pour objet un polypeptide ayant une activité uracile phosphoribosyl transférase (UPRTase), caractérisé en ce qu'il dérive d'une UPRTase native par délétion de la région N-terminale en amont du second codon ATG de ladite UPRTase native.

Au sens de la présente invention, un polypeptide ayant une activité UPRTase désigne un polypeptide capable de convertir l'uracile ou un de ses dérivés en un analogue monophosphaté et, en particulier la 5-FU en 5-FUMP.

L'UPRTase native dont dérive le polypeptide selon l'invention peut être d'une origine quelconque, notamment procaryotique, fongique ou de levure. A titre illustratif, les UPRTases d'*E. coli* (Anderson et al., 1992, Eur. J. Biochem *204*, 51-56), de *Lactococcus lactis* (Martinussen et Hammer, 1994, J. Bacteriol. *176*, 6457-6463), de *Mycobacterium bovis* (Kim et al., 1997. Biochem Mol. Biol. Int *41*, 1117-1124) et de *Bacillus subtilis* (Martinussen et al., 1995, J. Bacteriol. *177*, 271-274) peuvent être utilisées dans le cadre de l'invention. Mais on préfère tout particulièrement meure en oeuvre une UPRTase de levure et notamment celle codée par le gène *FUR1* de *S. cerevisiae* dont la séquence est divulguée dans Kern et al. (1990, Gene *88*, 149-157). A titre indicatif, les séquences des gènes et celles des UPRTases correspondantes peuvent être trouvées dans la littérature et les banques de données spécialisées (SWISSPROT, EMBL, Genbank, Medline...).

D'une manière générale, un polypeptide est constitué d'une partie N-terminale, d'une partie centrale et d'une partie C-terminale, chacune représentant environ le tiers de la molécule. Par exemple, l'UPRTase de *S. cerevisiae* ayant 251 acides aminés, sa partie N-terminale est constituée des 83 premiers résidus débutant à la méthionine dite initiatrice située en première position de la forme native. Quant à l'UPRTase d'*E. coli,* sa partie N-terminale couvre les positions 1 à 69.

Bien entendu, cette délétion peut être combinée à une ou plusieurs autres mutation(s) à un endroit quelconque de la molécule. De préférence. la ou les modifications supplémentaires n'affectent pas de manière significative les propriétés enzymatiques UPRTase du polypeptide selon l'invention. On indique que l'activité biologique des mutants peut être testée notamment par les techniques décrites dans les exemples qui suivent.

Par exemple, l'UPRTase codée par le gène *FUR1* comprend un premier codon ATG (codon ATG initiateur) en position + 1 suivi d'un second en position +36. Ainsi, la délétion des résidus +1 à 35 peut être envisagée dans le cadre de la présente invention, donnant un polypeptide débutant à la méthionine trouvée normalement en position +36 de la forme native.

Un polypeptide préféré selon l'invention comprend une séquence en acides aminés telle que représentée à l'identificateur de séquence IDS NO: 1, débutant au résidu Met en position 1 et se terminant au résidu Val en position 216.

Encore plus préférentiellement, il comprend la séquence en acides aminés représentée à l'identificateur de séquence IDS NO: 1. Comme mentionné ci-dessus, il peut comporter des mutations supplémentaires. On peut citer notamment la substitution du résidu sérine en position 2 (position 37 dans l'UPRTase native) par un résidu alanine.

Avantageusement, le polypeptide selon l'invention présente une activité UPRTase sensiblement plus élevée que celle présentée par ladite UPRTase native. Les résultats présentés dans les exemples qui suivent mettent clairement en évidence une conversion plus rapide et/ou plus efficace de la 5-FU en 5-FUMP, se traduisant par une cytotoxicité supérieure à l'égard des cellules transfectées ou transduites. Avantageusement, l'activité UPRTase du polypeptide selon l'invention est supérieure à celle présentée par l'UPRTase native d'un facteur 2 à 100, de préférence 5 à 75 et, de manière tout à fait préférée 10 à 50.

Selon un autre mode de réalisation, le polypeptide selon l'invention est un polypeptide de fusion dans lequel il est fusionné en phase avec au moins un second polypeptide. Bien que la fusion puisse avoir lieu à un endroit quelconque du premier polypeptide, les extrémités N ou C-terminales sont préférées et notamment l'extrémité N-terminale. Avantageusement, la fusion en phase met en oeuvre un second polypeptide présentant une activité cytosine désaminase (CDase) et dérivant d'une cytosine désaminase native, de sorte que le polypeptide de fusion selon l'invention présente les activités CDase et UPRTase. Une fusion FCY1::FUR1 (désignée ci-après FCU1) est préférée. Un tel polypeptide bifonctionnel permet d'améliorer la sensibilité des cellules cibles à la 5-FC et à la 5-FU. Par « activité cytosine désaminase », on entend couvrir la désamination de la cytosine ou d'un de ses analogues. De préférence, le second polypeptide selon l'invention est capable de métaboliser la 5-FC en 5-FU.

Dans le cadre de la présente invention, on a recours à une CDase d'origine procaryote ou eucaryote inférieur. Encore plus préférentiellement, il s'agit d'une CDase de levure et en particulier celle codée par le gène *FCY1* de *Saccharomyces cerevisiae*. Le clonage et la séquence des gènes codant pour les CDases de différentes sources sont disponibles dans la littérature et les banques de données spécialisées. On indique que la séquence du gène *FCY1* est divulguée dans Erbs et al. (1997, Curr. Genet. *31*, 1-6). Il est bien entendu possible d'utiliser un mutant de CDase ayant une capacité de conversion comparable ou supérieure à celle de l'enzyme native. L'homme de l'art est capable de cloner les séquences CDase à partir des données publiées, de procéder à d'éventuelles mutations, de tester l'activité enzymatique des formes mutantes dans un système acellulaire ou cellulaire selon la technologie de l'art ou en suivant le protocole indiqué ci-après et de fusionner en phase les polypeptides d'activité CDase et UPRTase.

Un exemple préféré est un polypeptide comprenant une séquence en acides aminés sensiblement telle que représentée à l'identificateur de séquence IDS NO: 2, débutant au résidu Met en position 1 et se terminant au résidu Val en position 373. Le terme « sensiblement » a la définition donnée précédemment. Un polypeptide comprenant la séquence en acides aminés telle que représentée à l'identificateur de séquence IDS NO: 2 convient tout particulièrement à la mise en oeuvre de l'invention.

Selon un mode de réalisation avantageux, un polypeptide bifonctionnel selon l'invention présente une activité CDase sensiblement plus élevée que ladite CDase native. En effet, les exemples qui suivent mettent en évidence que le couplage des deux enzymes permet une sensibilisation accrue à la 5-FC des cellules cibles. Le facteur d'augmentation est avantageusement d'au moins 2, de préférence d'au moins 5 et, de manière tout à fait préférée, de 10 ou plus.

D'une manière générale, un polypeptide selon l'invention peut être produit par les méthodes conventionnelles de synthèse chimique ou bien par les techniques de l'ADN recombinant (voir par exemple Maniatis et al., 1989, *Laboratory Manual*, Cold Spring Hàrbor, Laboratory Press, Cold Spring Harbor, NY). C'est pourquoi la présente invention couvre également un procédé de préparation selon lequel on introduit une séquence nucléotidique codant pour ledit polypeptide dans une cellule pour générer une cellule transformée, on cultive ladite cellule transformée dans des conditions appropriée pour permettre la production dudit polypeptide et on récolte ledit polypeptide à partir de la culture cellulaire. La cellule productrice peut être d'une origine quelconque et sans limitation, une bactérie, une levure ou bien une cellule de mammifère, dans la mesure où la séquence nucléotidique considérée est soit intégrée dans son génome soit intégrée dans un vecteur d'expression approprié capable de se répliquer. Bien entendu, la séquence nucléotidique est placée sous le contrôle de signaux de transcription et de traduction permettant son expression dans la cellule productrice. Vecteurs d'expression et signaux de contrôle sont connus de l'homme du métier. Quant au polypeptide, il peut étre récupéré du milieu ou des cellules (après lyse de celles-ci) et soumises à des étapes de purifications classiques (par chromatographie, électrophorèse, filtration, immunopurification etc...).

La présente invention concerne également une séquence nucléotidique codant pour un polypeptide selon l'invention. Il peut s'agir d'une séquence ADNc ou génomique ou de type mixte. Elle peut éventuellement contenir un ou plusieurs introns, ceux-ci étant d'origine native, hétérologue (par exemple l'intron du gène β-globine de lapin...) ou synthétiques afin d'augmenter l'expression dans les cellules hôtes. Comme déjà indiqué, ladite séquence peut coder pour un polypeptide dérivant de l'enzyme native ou un mutant présentant une activité comparable ou améliorée. Les séquences employées dans le cadre de la présente invention peuvent être obtenues par les techniques classiques de biologie moléculaire, par exemple par criblage de banque à l'aide de sondes spécifiques, par immunocriblage de banque d'expression, par PCR au moyen d'amorces adéquates ou par synthèse chimique. Les mutants peuvent être générés à partir des séquences natives par délétion et éventuellement substitution et/ou addition d'un ou plusieurs nucléotides en mettant en oeuvre les techniques de mutagénèse dirigée, de PCR, de digestion par les enzymes de restriction et ligation ou encore par synthèse chimique. La fonctionnalité des mutants et des constructions peut être vérifiée par le dosage de l'activité enzymatique ou par la mesure de la sensibilité de cellules cibles aux 5-FC et/ou 5-FU.

La présente invention a également trait à un vecteur recombinant portant une séquence nucléotidique selon l'invention placée sous le contrôle des éléments nécessaires à son expression dans une cellule hôte. Le vecteur recombinant peut être d'origine plasmidique, viral et éventuellement associé à une ou plusieurs substances améliorant l'efficacité transfectionnelle et/ou la stabilité du vecteur. Ces substances sont largement documentées dans la littérature accessible à l'homme de l'art (voir par exemple Felgner et al., 1987, Proc. West. Pharmacol. Soc. *32*, 115-121 ; Hodgson et Solaiman , 1996, Nature Biotechnology *14*, 339-342 ; Remy et al., 1994, Bioconjugate Chemistry *5*, 647-654). A titre illustratif mais non limitatif, il peut s'agir de polymères, de lipides notamment cationiques, de liposomes, de protéines nucléaires ou encore de lipides neutres. Ces substances peuvent être utilisées seules ou en combinaison. Une combinaison envisageable est un vecteur recombinant plasmidique associé à des lipides cationiques (DOGS, DC-CHOL, spermine-chol, spermidine-chol etc...) et des lipides neutres (DOPE).

Le choix des plasmides utilisables dans le cadre de la présente invention est vaste. Il peut s'agir de vecteurs de clonage et/ou d'expression. D'une manière générale, ils sont connus de l'homme de l'art et nombre d'entre eux sont disponibles commercialement mais il est également possible de les construire ou les modifier par les techniques de manipulation génétique. On peut citer à titre d'exemples les plasmides dérivés de pBR322 (Gibco BRL), pUC (Gibco BRL), pBluescript (Stratagène), pREP4, pCEP4 (Invitrogene) ou encore p Poly (Lathe et al., 1987, Gene *57*, 193-201). De préférence, un plasmide mis en oeuvre dans le cadre de la présente invention contient une origine de réplication assurant l'initiation de la réplication dans une cellule productrice et/ou une cellule hôte (par exemple, on retiendra l'origine ColE1 pour un plasmide destiné à être produit dans *E*. *coli* et le système oriP/EBNA1 si l'on désire qu'il soit autoreplicatif dans une cellule hôte mammifère, Lupton et Levine, 1985, Mol. Cell. Biol. *5*, 2533-2542 ; Yates et al., Nature *313*, 812-815). Il peut en outre comprendre un gène de sélection permettant de sélectionner ou identifier les cellules transfectées (complémentation d'une mutation d'auxotrophie, gène codant pour la résistance à un antibiotique...). Bien entendu, il peut comprendre des éléments supplémentaires améliorant son maintien et/ou sa stabilité dans une cellule donnée (séquence *cer* qui favorise le maintien monomérique d'un plasmide (Summers et Sherrat, 1984, Cell *36*, 1097-1103, séquences d'intégration dans le génome cellulaire).

S'agissant d'un vecteur viral, on peut envisager un vecteur dérivant d'un poxvirus (virus de la vaccine, notamment MVA, canaripox... etc), d'un adénovirus, d'un rétrovirus, d'un virus de l'herpès, d'un alphavirus, d'un foamyvirus ou d'un virus associé à l'adénovirus. On aura de préférence recours à un vecteur non réplicatif et non intégratif. A cet égard, les vecteurs adénoviraux conviennent tout particulièrement à la mise en oeuvre de la présente invention.

Les rétrovirus ont la propriété d'infecter et de s'intégrer majoritairement dans les cellules en division et à cet égard sont particulièrement appropriés pour l'application cancer. Un rétrovirus recombinant selon l'invention comporte généralement les séquences LTR, une région d'encapsidation et la séquence nucléotidique selon l'invention placée sous le contrôle du LTR rétroviral ou d'un promoteur interne tels que ceux décrits ci-après. Il peut dériver d'un rétrovirus d'une origine quelconque (murin, primate, félin, humain etc...) et en particulier du MoMuLV (Moloney murine leukemia virus), MVS (Murine sarcoma virus) ou Friend murine retrovirus (Fb29). Il est propagé dans une lignée d'encapsidation capable de fournir *en trans* les polypeptides viraux gag, pol et/ou env nécessaires à la constitution d'une particule virale. De telles lignées sont décrites dans la littérature (PA317, Psi CRIP GP + Am-12 etc...). Le vecteur rétroviral selon l'invention peut comporter des modifications notamment au niveau des LTR (remplacement de la région promotrice par un promoteur eucaryote) ou de la région d'encapsidation (remplacement par une région d'encapsidation hétérologue, par exemple de type VL30) (voir les demandes françaises FR 2 722 208 AB et FR 2 762 615 AB).

On aura de préférence recours à un vecteur adénoviral dépourvu de tout ou partie d'au moins une région essentielle à la réplication sélectionnée parmi les régions E1, E2, E4 et L1-L5, afin d'éviter sa propagation au sein de l'organisme hôte ou l'environnement. Une délétion de la région E1 est préférée. Mais elle peut être combinée à d'autres modification(s)/délétion(s) touchant notamment tout ou partie des régions E2. E4 et/ou L1-L5, dans la mesure où les fonctions essentielles défectives sont complémentées en *trans* au moyen d'une lignée de complémentation et/ou d'un virus auxiliaire. A cet égard, on peut avoir recours aux vecteurs de seconde génération de l'état de la technique (voir par exemple les demandes internationales WO-A-94/28152 et WO-A-97/04119). A titre illustratif, la délétion de la majorité de la région E1 et de l'unité de transcription E4 est tout particulièrement avantageuse. Dans le but d'augmenter les capacités de clonage, le vecteur adénoviral peut en outre être dépourvu de tout ou partie de la région E3 non essentielle. Selon une autre alternative, on peut mettre en oeuvre un vecteur adénoviral minimal retenant les séquences essentielles à l'encapsidation, à savoir les ITRs (Inverted Terminal Repeat) 5' et 3' et la région d'encapsidation. Les différents vecteurs adénoviraux ainsi que leurs techniques de préparation sont connus (voir par exemple Graham et Prevect, 1991, in *Methods in Molecular Biology,* vol 7, p 109-128 ; Ed : E.J. Murey, The Human Press Inc).

Par ailleurs, l'origine du vecteur adénoviral selon l'invention, peut être variée aussi bien du point de vue de l'espèce que du sérotype. Il peut dériver du génome d'un adénovirus d'origine humaine ou animale (canine, aviaire, bovine, murine, ovine, porcine, simienne...) ou encore d'un hybride comprenant des fragments de génome adénoviral d'au moins deux origines différentes. On peut citer plus particulièrement les adénovirus CAV-1 ou CAV-2 d'origine canine, DAV d'origine aviaire ou encore Bad de type 3 d'origine bovine (Zakharchuk et al., Arch. Virol., 1993, *128*: 171-176 ; Spibey et Cavanagh, J. Gen. Virol., 1989, 70: 165-172 ; Jouvenne et al., Gene, 1987, 60: 21-28 ; Mittal et al., J. Gen. Virol., 1995, 76: 93-102). Cependant, on préférera un vecteur adénoviral d'origine humaine dérivant de préférence d'un adénovirus de sérotype C, notamment de type 2 ou 5.

Un vecteur adénoviral selon la présente invention peut être généré *in vitro* dans *Escherichia coli* (*E. coli*) par ligation ou recombinaison homologue (voir par exemple la demande internationale WO-A-96/17070) ou encore par recombinaison dans une lignée de complémentation.

Les éléments nécessaires à l'expression sont constitués par l'ensemble des éléments permettant la transcription de la séquence nucléotidique en ARN et la traduction de l'ARNm en polypeptide. Ces éléments comprennent notamment un promoteur qui peut être régulable ou constitutif. Bien entendu, le promoteur est adapté au vecteur retenu et à la cellule hôte. On peut citer, à titre d'exemples, les promoteurs eucaryotes des gènes PGK (Phospho Glycérate Kinase), MT (metallothioneine ; Mc Ivor et al., 1987, Mol. Cell Biol. 7, 838-848), α-1 antitrypsine, CFTR, surfactant, immunoglobuline, β-actine (Tabin et al., 1982, Mol. Cell Biol. 2, 426-436), SRα (Takebe et al., 1988, Mol. Cell. Biol. 8, 466-472), le promoteur précoce du virus SV40 (Simian Virus), le LTR du RSV (Rous Sarcoma Virus), le promoteur TK-HSV-1, le promoteur précoce du virus CMV (Cytomegalovirus), les promoteurs du virus de la vaccine p7.5K pH5R, pK1L, p28, p11 et les promoteurs adénoviraux E1A et MLP. Il peut également s'agir d'un promoteur stimulant l'expression dans une cellule tumorale ou cancéreuse. On peut citer notamment les promoteurs des gènes MUC-1 surexprimé dans les cancers du sein et de la prostate (Chen et al., 1995, J. Clin. Invest. *96,* 2775-2782), CEA (pour carcinoma embryonic antigen) surexprimé dans les cancers du colon (Schrewe et al., 1990, Mol. Cell. Biol. *10*, 2738-2748), tyrosinase surexprimé dans les mélanomes (Vile et al., 1993, Cancer Res. *53*, 3860-3864), ERBB-2 surexprimé dans les cancers du sein et du pancréas (Harris et al., 1994, Gene Therapy *1*, 170-175) et α-fétoprotéine surexprimée dans les cancers du foie (Kanai et al., 1997, Cancer Res. *57*, 461-465). Le promoteur précoce du Cytomégalovirus (CMV) est tout particulièrement préféré.

Les éléments nécessaires peuvent, en outre, inclure des éléments additionnels améliorant l'expression de la séquence nucléotidique selon l'invention ou son maintien dans la cellule hôte. On peut citer notamment les séquences introniques, séquences signal de sécrétion, séquences de localisation nucléaire, sites internes de réinitiation de la traduction de type IRES, séquences poly A de terminaison de la transcription, leaders tripartites et origines de réplication. Ces éléments sont connus de l'homme de l'art.

Le vecteur recombinant selon l'invention peut également comprendre un ou plusieurs autres gènes d'intérêt, ceux-ci peuvent être placés sous le contrôle des mêmes éléments de régulation (cassette polycistronique) ou d'éléments indépendants. On peut citer en particulier les gènes codant pour les interleukines IL-2, IL-4, IL-7, IL-10, IL-12, les interférons, le facteur de nécrose des tumeurs (TNF), les facteurs stimulateurs des colonies (CSF) et notamment le GM-CSF, les facteurs agissant sur l'angiogénèse (par exemple PAI-1 pour plasminogen activator inhibitor). Dans un mode de réalisation particulier, le vecteur recombinant selon l'invention comprend le gène d'intérêt codant pour l'IL-2 ou pour l'interféron γ (INFγ). On peut également envisager d'associer la séquence nucléotidique selon l'invention à d'autres gènes suicides tels que le gène TK de HSV-1, la ricine, la toxine cholérique ...etc.

La présente invention a également trait à une particule virale comprenant un vecteur recombinant selon l'invention. Une telle particule virale peut être générée à partir d'un vecteur viral selon toute technique conventionnelle dans le domaine de l'art. Sa propagation est effectuée dans une cellule de complémentation adaptée aux déficiences du vecteur. S'agissant d'un vecteur adénoviral, on aura par exemple recours à la lignée 293 établie à partir de cellules de rein embryonnaire humain, qui complémente efficacement la fonction E1 (Graham et al., 1977, J. Gen. Virol. *36*, 59-72), la lignée A549-E1 (Imler et al., 1996, Gene Therapy 3, 75-84) ou une lignée permettant une double complémentation (Yeh et al., 1996, J. Virol. *70*, 559-565 ; Krougliak et Graham, 1995, Human Gene Therapy 6, 1575-1586 ; Wang et al., 1995 Gene Therapy 2, 775-783 ; demande internationale WO 97/04119). On peut également employer des virus auxiliaires pour complémenter au moins en partie les fonctions défectives. Par cellule de complémentation, on entend une cellule capable de fournir *en trans* les facteurs précoces et/ou tardifs nécessaires à l'encapsidation du génome viral dans une capside virale pour générer une particule virale contenant le vecteur recombinant. Ladite cellule peut ne pas complémenter à elle seule toutes les fonctions défectives du vecteur et dans ce cas peut être transfectée / transduite par un vecteur / virus auxiliaire apportant les fonctions complémentaires.

L'invention concerne également un procédé de préparation d'une particule virale, selon lequel :
(i) on introduit un vecteur recombinant selon l'invention dans une cellule de complémentation capable de complémenter *en trans* ledit vecteur, de manière à obtenir une cellule de complémentation transfectée,
(ii) on cultive ladite cellule de complémentation transfectée dans des conditions appropriées pour permettre la production de ladite particule virale, et
(iii) on récupère ladite particule virale dans la culture cellulaire.

Bien entendu, la particule virale peut être récupérée du surnageant de culture mais également des cellules. Une des méthodes couramment employée consiste à lyser les cellules par des cycles consécutifs de congélation / décongélation pour recueillir les virions dans le surnageant de lyse. Ceux-ci peuvent être amplifiés et purifiés selon les techniques de l'art (procédé chromatographique, ultracentrifugation notamment à travers un gradient de chlorure de césium...).

La présente invention concerne également une cellule hôte comprenant une séquence nucléotidique, un vecteur recombinant selon l'invention ou infectée par une particule virale selon l'invention. Aux fins de la présente invention, une cellule hôte est constituée par toute cellule transfectable par un vecteur recombinant-ou infectable par une particule virale, tels que définis ci-avant. Une cellule de mammifère et notamment humaine convient tout particulièrement. Elle peut comprendre ledit vecteur sous forme intégrée dans le génome ou non (épisome). Il peut s'agir d'une cellule primaire ou tumorale d'une origine quelconque, notamment hématopoïétique (cellule souche totipotente, leucocyte, lymphocyte, monocyte ou macrophage...), musculaire (cellule satellite, myocyte, myoblaste, muscle lisse...), cardiaque, pulmonaire, trachéale, hépatique, épithéliale ou fibroblaste.

La présente invention a également pour objet une composition comprenant un polypeptide. une séquence nucléotidique, un vecteur recombinant, une particule virale ou une cellule hôte selon l'invention en association avec un véhicule acceptable d'un point de vue pharmaceutique.

La présente invention concerne également une composition comprenant un polypeptide selon l'invention présentant une activité UPRTase et un autre polypeptide d'intérêt, notamment un polypeptide de l'art antérieur présentant une activité CDase.

La présente invention concerne encore une composition comprenant un polypeptide selon l'invention et un polypeptide d'intérêt codé par un des gènes d'intérêt mentionnés précédemment. Parmi ces polypeptides d'intérêt, on peut citer en particulier les interleukines IL-2, IL-4, IL-7, IL-10, IL-12, les interférons, le facteur de nécrose des tumeurs (TNF), les facteurs stimulateurs des colonies (CSF) et notamment le GM-CSF, les facteurs agissant sur l'angiogénèse (par exemple PAI-1 pour plasminogen activator inhibitor). L'IL-2 ou l'INFγ sont tout particulièrement envisagés.

La composition peut également être à base de séquences nueléotidiques permettant l'expression au sein de la cellule hôte des polypeptides précédents. Les séquences nucléotidiques peuvent être portées par un même vecteur d'expression ou par deux vecteurs indépendants. Bien entendu, ladite composition peut comprendre des particules virales générées à partir d'un ou des vecteurs viraux exprimant la (les) dite(s) séquence(s) nucléotidique(s).

C'est pourquoi la présente invention concerne également une composition comprenant une séquence nucléotidique selon l'invention codant pour un polypeptide présentant une activité UPRTase et une seconde séquence nucléotidique d'intérêt, codant notamment pour un polypeptide présentant une activité CDase.

La présente invention concerne encore une composition comprenant une séquence nucléotidique selon l'invention et une seconde séquence nucléotidique d'intérêt codant pour un polypeptide choisi parmi l'IL-2 et l'INFγ.

Une composition selon l'invention est plus particulièrement destinée au traitement préventif ou curatif de maladies par thérapie génique et s'adresse plus particulièrement aux maladies prolifératives (cancers, tumeurs, resténose... etc) et aux maladies d'origine infectieuse, notamment virale (induites par les virus de l'hépatite B ou C, le HIV, l'herpès, les rétrovirus....etc).

Une composition selon l'invention peut être fabriquée de manière conventionnelle en vue d'une administration par voie locale, parentérale ou digestive. En particulier, on associe une quantité thérapeutiquement efficace de l'agent thérapeutique ou prophylactique à un support acceptable d'un point de vue pharmaceutique. Les voies d'administration envisageables sont multiples. On peut citer par exemple la voie intragastrique, sous-cutanée, intracardiaque, intramusculaire, intraveineuse, intrapéritonéale. intratumorale, intranasale, intrapulmonaire ou intratrachéale. Pour ces trois derniers modes de réalisation, une administration par aérosol ou instillation est avantageuse. L'administration peut avoir lieu en dose unique ou répétée une ou plusieurs fois après un certain délai d'intervalle. La voie d'administration et le dosage appropriés varient en fonction de divers paramètres, par exemple, de l'individu, de la maladie à traiter ou encore du ou des gène(s) d'intérêt à transférer. Les préparations à base de particules virales selon l'invention peuvent être formulées sous forme de doses comprises entre 10⁴ et 10¹⁴ ufp (unités formant des plages), avantageusement 10⁵ et 10¹³ ufp et, de préférence, 10⁶ et 10¹² ufp. Pour ce qui est du vecteur recombinant selon l'invention, des doses comprenant de 0,01 à 100 mg d'ADN, de préférence 0,05 à 10 mg et, de manière tout à fait préférée, 0,5 à 5 mg peuvent être envisagées. Une composition à base de polypeptides comprend de préférence de 0,05 à 10 g et. de manière tout à fait préférée, de 0,5 à 5 g dudit polypeptide. Bien entendu, les doses peuvent être adaptées par le clinicien.

La formulation peut également inclure un diluant, un adjuvant ou un excipient acceptable d'un point de vue pharmaceutique, de même que des agents de solubilisation, de stabilisation, de préservation. Pour une administration injectable, on préfère une formulation en solution aqueuse, non-aqueuse ou isotonique. Elle peut être présentée en dose unique ou en multidose sous forme liquide ou sèche (poudre, lyophilisat...) susceptible d'être reconstituée de manière extamporanée par un diluant approprié. Elle peut également comprendre des quantités appropriées de prédrogues.

La présente invention est également relative à l'utilisation thérapeutique ou prophylactique d'un polypeptide, d'un vecteur recombinant, d'une particule virale ou d'une cellule hôte selon l'invention pour la préparation d'un médicament destiné au traitement du corps humain ou animal par thérapie génique ou par administration de protéine produite par voie recombinante. Selon une première possibilité, le médicament peut être administré directement *in vivo* (par exemple par injection intraveineuse, dans une tumeur accessible, dans les poumons par aérosol, dans le système vasculaire au moyen d'une sonde appropriée...). On peut également adopter l'approche *ex vivo* qui consiste à prélever des cellules du patient (cellules souches de la moelle osseuse, lymphocytes du sang périphérique, cellules musculaires...), de les transfecter ou infecter *in vitro* selon les techniques de l'art et de les réadminister au patient. Une utilisation préférée consiste à traiter ou prévenir les cancers, tumeurs et maladies résultant d'une prolifération cellulaire non désirée. Parmi les applications envisageables, on peut citer les cancers du sein, de l'utérus (notamment ceux induits par les papillomas virus), de la prostate, du poumon, de la vessie, du foie, du colon, du pancréas, de l'estomac, de l'oesophage, du larynx du système nerveux central et du sang (lymphomes, leucémie etc...). Elle est également utile dans le cadre des maladies cardiovasculaires, par exempte pour inhiber ou retarder la prolifération des cellules de muscles lisses de la paroi vasculaire (resténose). Enfin pour ce qui est des maladies infectieuses, l'application au SIDA peut être envisagée.

Lorsque l'utilisation met en oeuvre une séquence nucléotidique, un vecteur recombinant ou une particule virale permettant l'expression d'un polypeptide selon l'invention ayant une activité UPRTase, il peut être avantageux d'administrer en outre une seconde séquence nucléotidique codant pour un second polypeptide présentant une activité CDase, ladite seconde séquence nucléotidique étant portée par ledit vecteur recombinant ou particule virale ou par un vecteur ou une particule virale indépendante. Dans ce dernier cas, l'administration des séquences UPRTase et CDase peut être simultanée uu consécutive, l'ordre d'administration étant sans importance.

Selon un mode de réalisation avantageux, l'utilisation thérapeutique comprend également une étape supplémentaire selon laquelle on administre à l'organisme ou la cellule hôte des quantités acceptables d'un point de vue pharmaceutique d'une prédrogue, avantageusement d'un analogue de cytosine et, en particulier de la 5-FC. A titre illustratif, une dose de 50 à 500 mg/kg/jour peut être employée avec une préférence pour 200 mg/kg/jour. Dans le cadre de la présence invention, la prédrogue est administrée selon les pratiques standards et ceci de manière préalable, concomitante ou encore postérieure à celle de l'agent thérapeutique selon l'invention. La voie orale est préférée. On peut administrer une dose unique de prédrogue ou des doses répétées pendant un temps suffisamment long pour permettre la production du métabolite toxique au sein de l'organisme ou de la cellule hôte.

Par ailleurs, la composition ou la méthode selon l'invention peut être associée à une ou plusieurs substances potentialisant l'effet cytotoxique du 5-FU. On peut citer en particulier, les drogues inhibant les enzymes de la voie de biosynthèse *de novo* des pyrimidines (par exemple celles citées ci-après), les drogues telles que la Leucovorin (Waxman et al., 1982. Eur. J. Cancer Clin. Oncol. *18*, 685-692) qui en présence du produit du métabolisme du 5-FU (5-FdUMP) augmente l'inhibition de la thymidylate synthase ce qui entraîne une diminution du pool de dTMP nécessaire à la réplication et enfin les drogues telles que le méthotrèxate (Cadman et al., 1979, Science *250*, 1135-1137) qui en inhibant la dihydrofolate réductase et en élevant le pool de PRPP (phosphoribosylpyrophosphate) provoque l'augmentation d'incorporation de 5-FU dans l'ARN cellulaire.

La présente invention vise également l'utilisation des séquences ou vecteurs recombinants selon l'invention à titre de marqueur de sélection positive dans les cellules de mammifère. Avantageusement, les cellules sont transfectées puis le mélange cellulaire est cultivé en présence d'inhibiteurs de la voie de biosynthèse *de novo* des pyrimidines, tels que le PALA (N-(phosphonacétyl)-L-aspartate ; Moore et al., 1982, Biochem. Pharmacol. *31*, 3317-3321), le A77 1726 (métabolite actif du Leflunomide ; Davis et al., 1996, Biochem. *35*, 1270-1273) et le Brequinar (Chen et al., 1992, Cancer Res. *52*, 3251-3257). La présence de tels inhibiteurs bloque la synthèse *de novo* d'UMP nécessaire à la synthèse d'ARN et d'ADN avec pour conséquence la mort cellulaire. Cet effet cytotoxique peut être circonvenu par l'expression de la séquence nucléotidique selon l'invention codant pour une activité UPRTase en présence d'uracile ou par la co-expression de cette dernière avec les séquences codant pour une activité CDase (éventuellement sous forme fusionnée) en présence de cytosine. En conséquence, seules les cellules transfectées (ayant incorporées les séquences UPRTase/CDase) seront aptes à pousser en présence d'inhibiteurs de la voie de synthèse des pyrimidines. Ainsi, l'utilisation selon l'invention permet d'identifier et/ou isoler efficacement une cellule transfectée dans un mélange cellulaire.

La présente invention concerne également l'utilisation des séquences ou vecteurs recombinants selon l'invention à titre de marqueur de sélection négative dans les expériences d'interruption génique (« knock out » en anglais) des cellules souches embryonnaires qui sont à la base des procédés de préparation d'animaux transgéniques (voir par exemple Capecchi, 1989, Science 244, 1288-1292 ; Reid et al., 1990, Proc. Natl. Acad. Sci. USA 87, 4299-4303). Une telle utilisation, en association avec le gène de résistance à la néomycine par exemple peut permettre la sélection des cellules ayant subi un événement de recombinaison homologue qui seules seront aptes à pousser en présence de Généticine et des pyrimidines fluorés correspondants (5-FU si l'on met en oeuvre une séquence nucléotidique selon l'invention codant pour une activité UPRTase et 5-FC lorsque l'on met également en oeuvre une séquence nucléotidique codant pour une activité CDase). Les cellules ayant subi un événement de recombinaison non ciblé peuvent pousser en présence de Généticine mais pas des pyrimidines fluorés. Une autre utilisation potentielle à titre de marqueur de sélection négative se situe dans le domaine des plantes puisque tout comme les cellules mammifères, elles ne possèdent pas d'activité CDase endogène. Elles peuvent être sensibilisées à la 5-FC par transfection d'une séquence nucléotidique selon l'invention permettant l'expression d'une CDase exogène (voir par exemple Perera et al., 1993, Plant. Mol. Biol. 23, 793-799).

### EXEMPLES

La présente invention est illustrée, sans pour autant être limitée, par les exemples suivants dans lesquels :
- la figure 1 représente le taux de survie de souris B6D2 implantées avec des cellules tumorales B16F0. Les différents groupes de souris (10 souris par groupe) sont traitées par les compositions suivantes :
   - Vehicle/saline ; + Vehicle/5-FC ; * Ad null/saline ; - Ad null/5-FC; x ADFCU1/saline ; ◆ AdFCU1/5-FC
- la figure 2 représente le taux de survie de souris B6D2 implantées avec des cellules tumorales B16F0 . Les différents groupes de souris (10 souris par groupe) sont traitées par les compositions suivantes :
   - Vehicle/saline ; + Vehicle/GCV ; * Ad null/saline ; - Ad null/GCV ; x AdTK/saline ; ◆ AdTK/GCV
- la figure 3 représente le taux de survie de souris B6D2 implantées avec des cellules tumorales B16F0. Les différents groupes de souris (13 souris par groupe) sont traitées par les compositions suivantes :
   - Vehicle/saline ; + Vehicle/5-FC ; * Ad null/saline ; - Ad null/5-FC ; x Adnull + AdFCU1/saline ; ◆ Adnull + AdFCU1/5-FC ; ▼ Adnull + AdmuIFNg/saline ; Δ Adnull + AdmuIFNg/5-FC ; 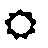 AdmuIFNg + AdFCU1/saline ; ○ AdmuIFNg + AdFCU1/5-FC
- la figure 4 représente le taux de survie de souris B6D2 implantées avec des cellules tumorales B16F0. Les différents groupes de souris (13 souris par groupe) sont traitées par les compositions suivantes :
   - Vehicle/saline ; + Vehicle/5-FC ; * Ad null/saline ; - Ad null/5-FC ; x Adnull + AdFCU1/saline ; ◆ Adnull + AdFCU1/5-FC ; ▼ Adnull + AdhuIL-2/saline ; Δ Adnull + AdhuIL-2/5-FC ; 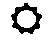 AdhuIL-2 + AdFCU1/saline ; ○ AdhuIL-2 + AdFCU1/5-FC

Les constructions décrites ci-dessous sont réalisées selon les techniques générales de génie génétique et de clonage moléculaire, détaillées dans Maniatis et al., (1989, Laboratory Manual, Cold Spring Harbor, Laboratory Press, Cold Spring Harbor, NY) ou selon les recommandations du fabricant lorsqu'on utilise un kit commercial. Les étapes de recombinaison homologue sont de préférence réalisées dans la souche *E. coli* BJ 5183 (Hanahan, 1983, J. Mol. Biol. *166*, 557-580). S'agissant de la réparation des sites de restriction, la technique employée consiste en un remplissage des extrémités 5' protubérantes à l'aide du grand fragment de l'ADN polymérase I *d'E. coli* (Kienow). Par ailleurs, les fragments de génome adénoviral employés dans les différentes constructions décrites ci-après, sont indiqués précisément selon leur position dans la séquence nucléotidique du génome de l'Ad5 telle que divulguée dans la banque de données Genebank sous la référence M73260.

En ce qui concerne la biologie cellulaire, les cellules sont transfectées ou transduites et cultivées selon les techniques standards bien connues de l'homme du métier. Pour ce qui est des levures. on utilise les techniques conventionnelles, notamment décrites dans Guthrie et Fink (1991, *Methods in Enzymology*, Vol 194, Guide to Yeast genetics and Molecular Biology, Academic Press Inc).

### EXEMPLE 1 : Construction du gène tronqué FUR1Δ105.

Le fragment *Eco*RI-*Hin*dIII portant le gène *FUR1* sauvage est isolé du plasmide pHX (Kern et al., 1990, Gene 88, 149-157) et inséré dans le plasmide intégratif levurien pRS306 (Sikorski et Hieter, 1989, Genetics *122*, 19-27) digéré par ces mêmes enzymes, pour donner le plasmide pRS306FUR1. A partir de ce dernier, le gène FUR1 est recloné sous forme d'un fragment *Xho*I-*Eco*RI dans le plasmide centromérique levurien pRS314 (Sikorski et hieter, 1989, Genetics *122*, 19-27) traité par *Xho*I et *Eco*RI. On obtient le phagemide pRS314FUR1 à partir duquel on prépare du simple brin uracilé dans la souche *E. coli* CJ236 (*dut, ung, thi, relA*, pCJ105 (Cm^{R}) sur lequel on procède à une mutagenèse dirigée selon la technique de Kunkel (kit Muta-Gene phagemid, In vitro Mutagenesis Version 2 ; Biorad). La mutagénèse a pour but d'introduire des sites de restriction *Eco*RI et *Mlu*I respectivement en 5' de l'ATG initiateur et 3' du codon stop du gène *FUR1* et de modifier le contexte de l'ATG initiateur selon les règles de Kozak, ce qui entraîne le changement du second codon Asn en Asp. Pour ce faire, on utilise les oligonucléotides reportés dans les IDS NO: 3 et 4. Le plasmide ainsi généré pRS314FUR1 + 1 est utilisé à titre de contrôle de l'activité UPRTase sauvage.

En parallèle, la délétion des 105 premiers nt de la séquence codante *FUR1* est obtenue par mutagénèse du plasmide pRS314FUR1 à l'aide des oligonucléotides reportés dans les IDS NO: 5 et 4. Le plasmide en résultant pRS314FUR1 + 106 comporte les mêmes modifications que son équivalent complet, à savoir un site *Eco*RI en 5' du codon ATG (correspondant à la Met +36 de la protéine native), un consensus Kozak à ce niveau avec un changement du codon suivant Ser en Ala et un site *Mlu*I en 3' du codon stop. Le fragment *Eco*RI-*Sac*I de pRS314FUR1 + 106 est recloné dans le plasmide pRS314FUR1 + 1 clivé par *Eco*RI et *Sac*I pour donner le plasmide pRS314FUR1Δ105 portant un gène *FUR1* exprimant une protéine délétée de ses 35 premiers résidus en N-terminal.

### EXEMPLE 2 : Complémentation d'un mutant fur1 de S. cerevisiae

La fonctionnalité des produits d'expression *FUR1* obtenus des constructions précédentes est évaluée par complémentation d'un mutant de *S. cerevisiae fur1-8 trp1-4* (Jund et Lacroute, 1970. J. Bacteriol. *102,* 607-615) transformé par les différents plasmides de type pRS314. Les transformants sont cultivés soit classiquement (milieu minimum YNB) ou en présence de 5-FU à 5mM et on évalue leur capacité de croissance à 30°C et 37°C. Les résultats sont présentés dans le tableau 1 où + représente la croissance et - l'absence de croissance témoignant d'une sensibilité au 5-FU et donc d'une activité UPRTase.

**Tableau 1**

| Transformants | Milieu YNB (minimum) 30°C et 37°C | +5-FU 5mM 30°C et 37°C |
|---|---|---|
| PRS314 | + | + |
| pRS314FUR1 | + | - |
| pRS314FUR1 + 1 | + | - |
| pRS314FUR1 + 106 | + | - |
| pRS314FUR1Δ105 | + | - |

Bien entendu, tous les transformants sont aptes à pousser en milieu conventionnel YNB. Par contre, en présence de 5-FU, seul le transformant comprenant le plasmide pRS314 « vide » ne comportant pas les séquences FUR1 est apte à croître alors que ceux ayant reçu les plasmides comportant les séquences FUR1 natives (pRS314FUR1), mutées au niveau du premier codon ATG (pRS314FUR1 + 1), mutées au niveau du second codon ATG (pRS314FUR1 + 106) ou tronquées (pRS314FUR1Δ105) sont sensibles à la présence de 5-FU. Ces tests de complémentation démontrent que les différentes constructions produisent une enzyme UPRTase fonctionnelle et que la délétion des 35 résidus N-terminaux n'a pas d'effet négatif sur l'activité enzymatique.

### EXEMPLE 3 : Expression de FUR1 et FUR1Δ105 dans les cellules mammifères.

Les fragments *Eco*RI-*Mlu*I de pRS314FUR1 + 1 et pRS314FUR1Δ105 portant les gènes *FUR1* et *FUR1Δ105* ont été introduits dans le plasmide d'expression eucaryote pCI-neo (Promega) pour donner respectivement les plasmides pCI-neoFUR1+1 et pCI-neoFUR1Δ105. Les cellules COS7 (ATCC CRL-1651) sont transfectées transitoirement par ces plasmides et on détermine l'activité enzymatique UPRTase sur les extraits cellulaires.

Plus précisément. 5x10⁵ cellules COS7 sont ensemencées à 37°C dans des boites de culture de 60 mm contenant 5ml de milieu DMEM complémenté par du sérum de veau fétal (FCS) 10%. Le jour suivant, les cellules sont traitées par 20 µl de lipofectine (Gibco BRL) en absence ou en présence de 5 µg de plasmide et la culture poursuivie dans 2 ml de milieu DMEM. Après 16 heures d'incubation à 37°C, elles sont remises dans 5 ml de DMEM-FCS 10% pendant environ 48 heures. Les cellules sont alors lavées et resuspendues dans 30 µl de tampon de lyse (Tris-HCl pH 7,5 50 mM / NaCl 150 mM / EDTA 5 mM / DTT 1 mM / triton X-100 1 %) pendant 30 minutes à 4°C. Le lysat cellulaire est recueilli, centrifugé et on dose l'activité enzymatique UPRTase sur le surnageant ainsi obtenu. Pour ce faire, 4 µl de lysat sont incubés 10 minutes à 37°C en présence de 2 µl de tampon réactionnel UPRTase (Tris-HCl pH 7,5 100 mM / MgCl₂ 10 mM / 5-PRPP 10 mM (5-phosphoribosylpyrophosphate) /[C¹⁴]uracile 3 mM à 0,02 µCi/µl (NEN). Les réactions enzymatiques sont stoppées par chauffage à 100°C pendant 1 minute. Des aliquotes de 1 µl sont déposés sur plaque TLC polyethylenimine-cellulose (Merck). L'uracile est séparé de l'UMP en utilisant l'eau comme solvant de migration. Après migration, la plaque TLC est scannée au Posphorlmager (445 SI ; Molecular Dynamics). La concentration en protéine est mesurée par la méthode de Bradford (protein Assay ; Biorad). L'activité UPRTase à été déterminée à partir de trois transfections indépendantes et les résultats présentés dans le Tableau 2.

**Tableau 2**

| Plasmide transfecté | Activité UPRTase (37°C) pmoles d'uracile transformées/minute/mg de protéine |
|---|---|
| - | non détectable |
| PCI-neo | non détectable |
| PCI-neoFUR1 + 1 | 91 +/- 15 |
| PCI-neoFUR1Δ105 | 3150 +/- 420 |

La transformation de l'uracile en UMP n'est observée que dans les cellules transfectées par les plasmides exprimant un gène *FUR1* complet (pCI-neoFUR1+1) ou tronqué (pCI-neoFUR1Δ105). On note également que l'activité UPRTase codée par le gène tronqué est supérieure d'un facteur 30 par rapport à celle codée par le gène complet.

### EXEMPLE 4 : Construction d'un vecteur adénoviral pour le transfert du gène tronqué FUR1Δ105 et infection de cellules tumorales humaines.

### 1. Construction de pTG6289 pour l'expression de FUR1Δ105

Le fragment *Eco*RI-*Mlu*I de pCI-neoFUR1Δ105 renfermant le gène tronqué est isolé et introduit dans le vecteur pTG6600 clivé par ces mêmes enzymes, pour donner le vecteur de transfert pTG6288. A titre indicatif, pTG6600 est un vecteur p polyII (Lathe et al., 1987, Gene *57*, 193-201) dans lequel sont insérés les séquences Ad5 1 à 458, le promoteur précoce CMV, les séquences d'épissage hybrides trouvées dans le plasmide pCI (Promega Corp, comprenant le site donneur d'épissage de l'intron 1 du gène β-globine humaine et le site accepteur d'épissage du gène d'immunoglobuline de souris), les séquences de polyadénylation du virus SV40 et les séquences Ad5 3328-5788. Une telle construction est à la portée de l'homme de l'art, notamment sur la base du document FR-A-2 763 959. Le vecteur adénoviral pTG6289 est reconstitué par recombinaison dans la souche *E. coli* BJ 5183 entre le fragment *Pac*I-*Bst*EII de pTG6288 et le vecteur pTG6624 (décrit dans le document FR-A-2 763 959) linéarisé par *Cla*I. A titre indicatif, pTG6624 correspond au plasmide p poly II portant le génome Ad5 délété des régions E1 (nt 459 à 3327) et E3 (nt 28592 à 30470) avec une cassette d'expression insérée à la place de E1.

La construction finale pTG6289 contient le génome Ad5 délété de l'essentiel des régions E1 (nt 459 à 3328) et E3 (nt 28249 à 30758) et en lieu et place de E1, une cassette d'expression du gène FUR1Δ105 placé sous le contrôle du promoteur précoce CMV et des séquences d'épissage hybride β-globine/Ig. Les particules adénovirales sont générées par transfection dans une lignée de complémentation de la fonction E1, par exemple la lignée 293 (ATCC CRL1573) selon les techniques de l'art (Graham et Prevec, 1991, Methods in Molecular Biology Vol7, Gene Transfer and Expression Protocols ; Ed E.J. Murray, The Human Press Inc, Clinton, NJ).

### 2. Construction d'un vecteur pour l'expression du gène FCY1.

On isole du simple brin uracilé à partir du phagemide d'expression pRS315FCY1 (Erbs et al., 1997. Curr. Genet. 31, 1-6), lequel est modifié par mutagénèse dirigée (kit Muta-gene Phagemid. In vitro Mutagenesis version 2 ; Biorad). La mutagénèse met en oeuvre les oligonucléotides des IDS NO: 6 et 7 permettant d'introduire un site *Hin*dIII en 5' de l'ATG initiateur et un site *Eco*RI en 3' du codon stop du gène *FCY1* et de modifier le contexte traductionnel selon les règles de Kozak.. Le fragment *Hin*dIII-*Eco*RI ainsi modifié est inséré dans le plasmide pRS306 (Sikorski et Hieter. 1989, Genetics 122, 19-27) digéré par ces mêmes enzymes, pour donner pRS306FCY1. Le fragment *Xho*I-*Xba*I de pRS306FCY1 est sous-cloné dans le vecteur pCI-neo (Promega) clivé par *Xho*I et *Xba*I. On obtient pcI-neoFCY1 comprenant le gène *FCY1* avec une séquence consensus Kozak au niveau de son ATG initiateur.

On génère un vecteur adénoviral pour l'expression du gène *FCY1* de *S. cerevisiae* codant pour l'enzyme CDase. Le fragment *Xho*I-*Xba*I du plasmide pCI-neoFCY1, renfermant le gène *FCY1* est introduit dans le vecteur pTG6600 digéré par *Xho*I-*Xba*I, pour donner pTG6286. Comme précédemment, la recombinaison homologue entre le fragment *Pac*I-*Bst*EII portant *FCY1* et isolé de pTG6286 et le vecteur pTG6624 linéarisé par *Cla*I permet de générer le vecteur adénoviral pTG6287 délété des régions E1 et E3 et comportant à la place de E1 le gène FCY1 placé sous le contrôle du promoteur CMV et des séquences d'épissage hybride β-globine/Ig. Les particules virales (AdTG6287) sont obtenues par transfection de cellules 293.

### 3. Détermination des activités enzymatiques.

Les constructions adénovirales sont testées sur trois lignées tumorales humaines: PANC-1 (carcinome de pancréas / ATCC CRL-1469), SK-BR-3 (adénocarcinome du sein / ATCC HTB-30) et SW480 (adénocarcinome de colon / ATCC CCL-228). Ces cellules ont été choisies à cause de leur résistance au 5-FU, permettant ainsi de déterminer l'efficacité du gène FUR1Δ105.

Plus précisément, 5x10⁶ cellules en suspension dans 100 µl de PBS-FCS 2% sont incubées 30 minutes à 37°C en présence des adénovirus à tester (Ad.CMV-FCY1, Ad.CMV-FUR1 ou leur mélange) à une MOI (multiplicité d'infection) de 20. A titre de témoin négatif, l'infection est réalisée avec du Mock ou un adénovirus de première génération dépourvu de gène d'intérêt (Ad null). Les cellules infectées sont reprises dans 500 µl de DMEM-FCS 10% et placées dans une boite de culture de 35 mm à raison de 1x10⁶ cellules par boite. Après 48 heures d'incubation à 37°C, les différentes activités enzymatiques utilisant la 5-FC ou la 5-FU comme substrat, sont déterminées sur les lysats cellulaires. L'activité UPRTase est mesurée selon le protocole indiqué ci-avant, sur 4 µl de lysat cellulaire incubés 20 minutes à 37°C en présence de 2 µl de tampon réactionnel UPRTase ([C¹⁴]5-FU, Sigma). Pour l'activité CDase, on utilise 4 µl de lysat cellulaire qui sont incubés 20 min à 37°C avec 2 µl de tampon réactionnel CDase (Tris-HCl pH 7,5 100 mM / [H³]5-FC 3 mM à 0.25 µCi/µl, Sigma). La conversion de la 5-FC en 5-FUMP (activités CDase/UPRTase) est déterminée sur 4 µl de lysat cellulaire incubés 20 min à 37°C en présence de 2 µl de tampon réactionnel CDase/UPRTase (Tris-HCl pH 7,5 100 mM / MgCl2 10 mM / 5-PRPP 10 mM / [H³]5-FC 3 mM à 0.25 µCi/µl). Des aliquotes de 1 µl sont déposés sur plaque TLC PEI cellulose. La 5-FU est séparée de la 5-FC en utilisant l'eau comme solvant et la séparation de la 5-FUMP et de la 5-FU est réalisée en utilisant un mélange eau/butanol-1 (14%/86%) comme solvant.

Dans les trois types cellulaires, l'activité CDase est observée dans les extraits obtenus des cellules infectées avec l'adénovirus exprimant le gène *FCY1* (Ad.CMV-FCY1) et de celles coinfectées avec les deux virus Ad.CMV-FCY1 et Ad.CMV-FUR1Δ105. Le dosage enzymatique montre une activité variant d'environ 80 à 100 pmoles de 5-FC transformée/min/mg de protéine dans les cellules PANC-1, SK-BR3 et SW480. Pour ce qui est de la transformation du 5-FU (activité UPRTase), on observe une activité endogène d'environ une centaine de p moles de 5-FU transformée/min/mg de protéine. L'activité est amplifiée d'un facteur 7 à 8 lorsque l'infection est réalisée avec l'adénovirus Ad.CMV-FUR1Δ105 seul ou dans le cas de la coinfection. Enfin, la conversion de 5-FC en 5-FUMP est mise en évidence dans les extraits infectés avec Ad-CMV-FCY1 (grâce à l'activité UPRTase endogène) et dans le cas des coinfections.

Par ailleurs on détermine les LD50 (dose létale 50 %) de la 5-FC et de la 5-FU (µM) vis-à-vis des trois lignées tumorales humaines infectées par les virus précédents (Mock, Ad null, Ad.CMV-FCY1, Ad.CMV-FUR1Δ105) ou coinfectées par les adénovirus exprimant les gènes *FCY1* et *FUR1Δ105*. Les cellules sont infectées à une MOI de 20 puis cultivées en présence ou en absence de drogues (5-FC ou 5-FU) à différentes concentrations. Après trypsination à J+6, on ajoute du bleu trypan. Les cellules vivantes ne prennent pas le bleu trypan alors que les cellules mortes sont colorées. On évalue au microscope le % de mortalité. Les valeurs reportées ci-après correspondent aux moyennes obtenues après quatre comptages.

La LD50 à la 5-FC des lignées cellulaires infectées est comprise entre 5 et 10 µM (selon les lignées) lorsqu'il y a co-expression des deux gènes *FCY1* et *FUR1Δ105*, alors qu'elle est de 100 à 500 µM dans le cas de la seule expression du gène FCY1 et de 5000 à 10000 µM pour les témoins négatifs (Mock, Ad null) et l'Ad.CMV-FUR1Δ105. De même, la LD50 à la 5-FU est considérablement abaissée lorsque l'infection met en oeuvre le virus permettant l'expression du gène *FUR1Δ105* comparée aux témoins négatifs et à l'infection mettant en oeuvre l'Ad.CMV-FCY1 seul. Ces résultats mettent en évidence l'intérêt d'utiliser en synergie les gènes *FCY1* et *FUR1Δ105* dans une approche gène « suicide » pour améliorer la conversion de la prédrogue 5-FC en 5-FUMP cytotoxique.

### EXEMPLE 5 : Construction d'un gène hybride codant pour une protéine de fusion portant les activités CDase et UPRTase

### 1. Construction du gène de fusion

Le fragment *Eco*RI*-Not*I de pCl-neoFUR1Δ105 est inséré dans le plasmide pCI-neoFCY1 coupé par *Eco*RI*-Not*I pour donner le plasmide pCI-neoFCY1+FUR1Δ105. A partir de ce plasmide simple brin uracilé, la fusion de *FCY1* et *FUR1Δ105* est réalisée par mutagenèse dirigée à l'aide de l'oligonucléotide oTG 11615 (IDS NO: 8). La modification consiste en une délétion du codon stop de *FCY1* et du codon Met initiateur de *FUR1* permettant la fusion en phase des séquences *FCY1* et *FUR1Δ105* sans espaceur (fusion désignée ci-après FCU1).

### 2. Caractérisation enzymatique de la protéine bifonctionnelle FCU1.

Les activités CDase et UPRTase (10 minutes de réaction à 37°C) sont déterminées selon les techniques indiquées au préalable à partir de lysats cellulaires de cellules COS7 :
- non transfectées
- transfectées par 10 µg de pCI-neo (plasmide vide)
- transfectées par 5 µg de pCI-neoFCY1 + 5 µg de pCI-neoFUR1Δ105
- transfectées par 5 µg de pCI-neo + 5 µg de pCI-neoFCU1

Les valeurs obtenues sont reportées dans le Tableau 3 suivant et correspondent aux moyennes de trois déterminations enzymatiques.

**Tableau 3**

| COS7 | **Cdase** pmoles de cytosine transformées/min/mg de protéine | **UPRTase** pmoles d'uracile transformées/min/mg de protéine | **Cdase/UPRTase** Pmoles d'UMP formées/min/mg de protéine |
|---|---|---|---|
| Non transfectées | non détectable | non détectable | non détectable |
| pCI-neo | non détectable | non détectable | non détectable |
| PCI-neoFCY1 + PCI-neoFUR1Δ105 | 626 +/- 106 | 3005 +/- 298 | 535 +/- 54 |
| pCI-neo + pCI-neoFCU1 | 5373 +/- 268 | 2789 +/-310 | 2121 +/- 107 |

On observe une activité UPRTase équivalente que le gène soit sous forme de fusion (pCI-neoFCU1) ou non (pCI-neoFUR1Δ105). De manière surprenante, l'activité CDase est augmentée d'un facteur 10 lorsque le gène est sous forme de fusion par rapport au gène *FCY1* non fusionné. Ainsi. la forme fusionnée est bénéfique puisqu'elle provoque une augmentation de l'activité globale CDase-UPRTase et donc de la formation de 5-FUMP à partir de 5-FC.

La protéine de fusion codée par *FCU1* présentant une activité UPRTase limitante par rapport à l'activité CDase, on met en évidence *in vitro* lors de la réaction CDase-UPRTase. la formation d'un pool d'uracile de l'ordre de 3000 pmoles d'uracile/min/mg de protéine. Ce pool d'uracile est inexistant dans les mêmes conditions lorsque la réaction met en oeuvre les protéines *FCY1* et *FUR1Δ105*.

### 3. Expression des gènes FCY1, FUR1Δ105 et FCU1 clonés sur plasmide pCI-neo, dans les lignées B16(F0).

L'efficacité du gène fusion *FCU1* est évaluée par transfection de la lignée de mélanome de souris B16(F0) (ATCC CRL-6322). Pour ce faire, 2,5x10⁵ cellules B16(F0) sont ensemencées à 37°C dans des boites de 60 mm contenant 5 ml de milieu DMEM-FCS 10%. Après 48 heures d'incubation. les cellules ont été traitées par 20 µl de lipofectine (Gibco BRL) en présence de 5 µg de plasmide et la culture poursuivie pendant 16 heures à 37°C dans 2 ml de milieu DMEM. 48 heures après, les cellules sont placées dans 5 ml de DMEM-FCS 10% puis diluées dans des boites de Pétri de 10 cm dans du milieu DMEM-FCS 10% contenant 1 mg/ml de G418. Au bout de 20 jours, des clones résistants au G418 sont visibles. Les clones pour chaque construction sont rassemblés, transférés dans des flasques T150 et cultivés dans le milieu sélectif. On détermine les activités CDase, UPRTase et CDase-UPRTase à 37°C en présence de 5-FC ou de 5-FU comme substrat. Les valeurs indiquées sont les moyennes obtenues après trois déterminations enzymatiques (voir Tableau 4).

**Tableau 4**

| B16(F0) | **CDase** pmoles de 5-FC transformées/min/mg de protéine | **UPRTase** pmoles de 5-FU transformées/min/mg de protéine | **CDase/UPRTase** pmoles de 5-FUMP formées/min/mg de protéine |
|---|---|---|---|
| non transfectées | non détectable | 310 +/- 77 | non détectable |
| pCI-neo | non détectable | 238 +/- 56 | non détectable |
| pCI-neoFCY1 | 3 +/- 0.4 | 292 +/- 93 | 2 +/- 0.4 |
| pCI-neoFUR1Δ105 | non détectable | 813 +/- 47 | non détectable |
| pCI-neoFCU1 | 158 +/- 9 | 834 +/-67 | 41 +/- 5 |

Les résultats enzymatiques confirment ceux déterminés à partir des cellules COS7 transfectées, à savoir une activité UPRTase conservée mais une activité CDase fortement augmentée pour la protéine de fusion par rapport celle mesurée avec la protéine native *FCY1*. Pour les clones B16 transfectés par pCI-neoFCU1, il apparaît également *in vitro* un pool de 5-FU (120 pmoles/min/mg de protéine) qui est la conséquence de l'augmentation de l'activité CDase.

Les sensibilités aux 5-FC et 5-FU des différents clones sont également testées après incubation en présence des drogues pendant 6 jours. Les valeurs suivantes sont les moyennes de quatre comptages (au bleu trypan) de la mortalité des cellules. La LD50 à la 5-FC est de 10 µM pour les cellules transfectées avec le plasmide portant le gène de fusion (pCI-neoFCU1) comparé à 100 µM avec pCl-neoFCY1 et 1000 µM avec les témoins négatifs (cellules non transfectées et pCI-neo) et pCI-neoFUR1Δ105. La LD50 à la 5-FU est de 0,03 µM lorsque la transfection est réalisée avec pCI-neoFUR1Δ105 ou pCI-neoFCU1 contre 0,05 µM dans tous les autres cas.

Ces résultats montrent que l'expression du gène *FCU1* sensibilise plus fortement les cellules au 5-FC que l'expression du gène *FCY1*. Cette différence de sensibilisation à la 5-FC est due à une activité CDase plus importante pour la protéine de fusion et non à la présence de l'activité UPRTase; cette activité UPRTase codée par le gène de *S. cerevisiae* a peu d'effet sur la toxicité à la 5-FU des B16(F0) qui sont naturellement très sensibles à la 5-FU et qui présentent une activité UPRTase endogène importante.

### 4. Mise en évidence d'un effet « bystander » des B16(F0) transfectées par le gène FCU1.

L'apparition d'un pool de 5-FU dans le dosage *in vitro* de l'activité CDase-UPRTase de B16(FO) transfectées par *FCU1* est *à priori* un avantage pour potentialiser l'effet « bystander » : plus ce pool intracellulaire est important, plus il y aura diffusion passive de la 5-FU dans le milieu, et plus grande sera la mortalité des cellules non transfectées.

Pour mettre en évidence cet effet « bystander », des clones B16(F0) non transfectés ont été mélangés, à différentes proportions, à des clones B16(F0) transfectés par pCI-neoFCU1. Ces mélanges de clones ont été par la suite testés en présence de différentes concentrations de 5-FC. On évalue la mortalité cellulaire (au bleu trypan) après 4 jours de culture en présence de 5-FC Les résultats (moyennes de 4 déterminations) sont présentés dans le Tableau 5 suivant.

**Tableau 5**

| B16/B16 pCl-neoFCU1 en % | **LD50** (µM) + **5-FC** |
|---|---|
| 100%/0% | 5000 |
| 95%/5% | 500 |
| 90%/10% | 300 |
| 75%/25% | 200 |
| 50%/50% | 100 |
| 0%/100% | 50 |

On observe que la LD50 à la 5-FC est abaissée d'un facteur 10 dès que le mélange cellulaire contient 5% de cellules tranfectées par *FCU1*, témoignant de l'existence d'un effet « bystander ». Bien entendu, la LD 50 continue à décroître lorsque la proportion de cellules transfectées augmente.

Dans le but de démontrer que cet effet « bystander » ne nécessite pas, à l'inverse du système *tk*/ganciclovir, un contact entre cellules grâce à la diffusion de la 5-FU dans le milieu, des cellules B16 (non transfectées) ont été incubées pendant 4 jours dans du milieu (dilué au quart dans du milieu neuf) provenant de surnageants de culture de B16 (témoin négatif) ou B16 pCI-neoFCU1 incubées 48 heures en présence de différentes concentrations de 5-FC et la mortalité des cellules B16 est évaluée au bleu de trypan. Les résultats démontrent un effet drastique sur la LD50 et mettent en évidence la présence de la 5-FU dans le milieu qui est la conséquence d'une forte activité CDase de la protéine fusion codée par le gène *FCU1*.

### EXEMPLE 6 Construction d'un vecteur adénoviral pour le transfert du gène FCU1 et infection de cellules tumorales.

### 1. Construction de pTG13060 pour l'expression de FCU1.

Le fragment *Xho*I-*Mlu*I de pCI-neoFCU1 renfermant le gène fusion *FCU1* est isolé et introduit dans le vecteur pTG6600 linéarisé par *Xho*I-*Mlu*I, pour donner le vecteur de transfert pTG 13059. La recombinaison homologue entre le fragment *Pac*I-*Bst*EII portant *FCU1* et isolé de pTG13059 et le vecteur pTG6624 linéarisé par *Cla*I permet de générer le vecteur adénoviral pTG13060 délété des régions E1 et E3 et comportant à la place de E1 le gène *FCU1* placé sous le contrôle du promoteur CMV et des séquences d'épissage hybrides β-globine/Ig. Les particules virales (Ad.CMV-FCU1) sont obtenues par transfection de cellules 293.

### 2. Expression dans les cellules tumorales du gène FCU1 cloné dans un vecteur adénoviral.

La nouvelle construction adénovirale est testée sur les trois lignées tumorales humaines: PANC-1, SK-BR-3 et SW480. Les activités CDase et UPRTase (en utilisant la 5-FC ou la 5-FU comme substrat) sont déterminées selon les techniques indiquées auparavant à partir de lysats cellulaires des cellules humaines infectées à une MOI de 20. On observe dans les trois lignées cellulaires infectées par Ad.CMV-FCU1 une activité UPRTase (de 700 à 800 pmoles de 5-FU transformées/min/mg de protéine) équivalente par rapport à l'activité UPRTase exprimée par le gène *FUR1Δ105*. Dans les trois types cellulaires, l'activité CDase est amplifiée d'un facteur 100 (10000 à 12000 pmoles de 5-FC transformées/min/mg de protéine) lorsque le gène est exprimé sous forme de fusion (*FCU1*) par rapport à l'activité des cellules infectées par Ad.CMV-FCY1.

La protéine de fusion exprimée à partir d'un vecteur adénoviral présente, comme dans l'exemple 5, une activité UPRTase limitante par rapport à l'activité CDase ce qui conduit à la formation d'un pool important de 5-FU de l'ordre de 10000 pmoles de 5-FU/min/mg de protéine lors de la réaction CDase-UPRTase. Cette accumulation de 5-FU permettant de potentialiser l'effet « bystander ».

On détermine les LD50 de la 5-FC et de la 5-FU vis-à-vis des trois lignées tumorales humaines infectées par les différents virus (Ad null, Ad.CMV-FCY1, Ad.CMV-FUR1Δ105 et Ad.CMV-FCU1) ou coinfectés par les adénovirus exprimant les gènes *FCY1* et *FUR1Δ105*. Les cellules sont infectées à une MOI de 5 puis cultivées en présence de différentes concentrations de 5-FC et de 5-FU. Après 6 jours de culture, la viabilité des cellules est déterminée au bleu trypan. Les valeurs de LD50 reportées dans le tableau 6 sont les moyennes de quatre comptages.

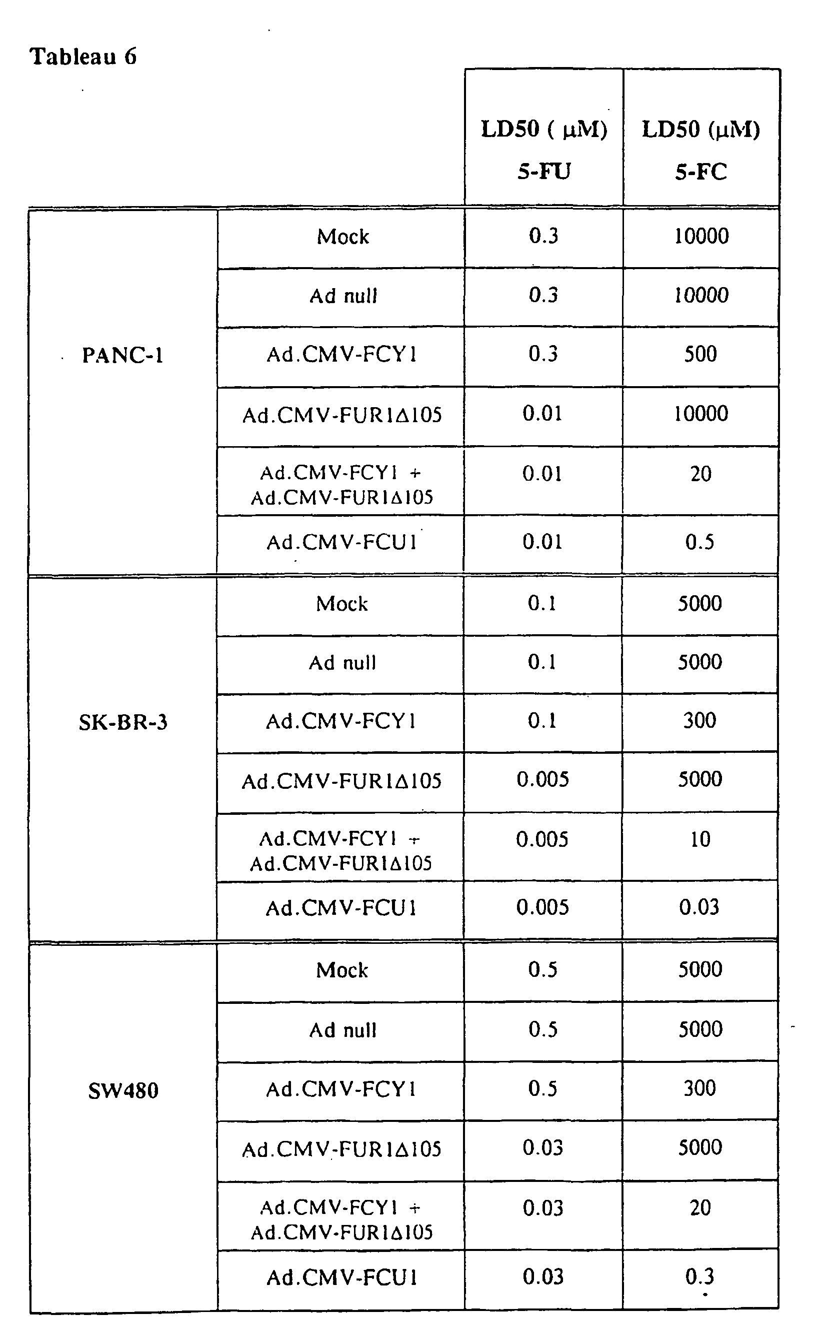

Ces résultats montrent que l'expression du gène *FCU1* à partir d'un vecteur adénoviral sensibilise plus fortement les cellules au 5-FC que la coexpression des gènes *FCY1* et *FUR1Δ105*. Cette plus grande sensibilité au 5-FC découle de l'augmentation de l'activité CDase codée par le gène fusion *FCU1*.

### 3. Mise en évidence d'un effet « bystander » des cellules tumorales humaines infectées par les différents adénovirus.

Les cellules tumorales humaines PANC-1, SK-BR-3 et SW480 sont infectées (Ad null, Ad.CMV-FCY1, Ad.CMV-FUR1Δ105 et Ad.CMV-FCU1) à une MOI de 20. Après 48 heures d'incubation à 37°C, les cellules sont rincées deux fois au PBS, trypsinisées et étalées à différentes proportions en présence de cellules non infectées. Après 6 jours de culture en présence de 1 mM de 5-FC, la mortalité cellulaire est évaluée au bleu trypan. Les résultats (moyenne de 4 déterminations) montrent pour les trois souches tumorales qu'un mélange de 10% de cellules infectées par Ad.CMV-FCY1 et 90% de cellules non infectées entraîne 100% de mortalité. Dans le cas des cellules infectées par Ad.CMV-FCU1. 1% de cellules infectées est suffisant pour entraîner 100% de mortalité. Cette expérience démontre l'existence d'un important effet « bystander » qui découle de l'augmentation du pool de 5-FU dans les cellules exprimant la protéine de fusion.

Dans le but de mettre en évidence l'excrétion dans le milieu de 5-FU par les cellules exprimant FCY1 et FCU1 en présence de 5-FC, 10⁶ cellules PANC-1, SK-BR-3 et SW480 sont infectées (Ad null. Ad.CMV-FCY1, Ad.CMV-FUR1Δ105 et Ad.CMV-FCU1) à une MOI de 20. Les cellules sont cultivées en présence de 1 mM de [³H]5-FC à 0.25 µCi/µl. Après 48 heures de culture, des aliquotes de 2 µl de milieu de culture sont déposés sur plaque TLC cellulose. La 5-FU est séparée de la 5-FC en utilisant un mélange eau/butanol-1 (14%/86%) comme solvant. Dans les trois types cellulaires, pour les cellules infectées par Ad.CMV-FCY1, environ 20% du 5-FC est converti en 5-FU. Pour les cellules infectées par Ad.CMV-FCU1, plus de 90% du 5-FC est transformé en 5-FU.

### 4. Comparaison de l'efficacité des systèmes FCU1/5-FC et TK/GCV.

Dans le but de comparer l'efficacité du système FCU1/5-FC par rapport au système TK/GCV, on génère un vecteur adénoviral pour l'expression du gène de la thymidine kinase du virus herpes simplex de type 1 (TK HSV-1). Le fragment *Eco*RI-*Xba*I de pTG4043 renfermant le cDNA de TK HSV-1 est isolé et introduit dans le vecteur pTG6600 linéarisé par *Eco*RI*-Xba*I, pour donner le vecteur de transfert pTG6222. La recombinaison homologue entre le fragment *Pac*I-*Bst*EII portant TK HSV-1 et isolé de pTG6222 et le vecteur pTG6624 linéarisé par *Cla*I permet de générer le vecteur adénoviral pTG13019 délété des régions E1 et E3 et comportant à la place de E1 le gène TK HSV-1 placé sous le contrôle du promoteur CMV et des séquences d'épissage hybrides β-globine/Ig. Les particules virales (Ad.CMV-TK) sont obtenues par transfection de cellules 293.

Les cellules humaines PANC-1, SK-BR-3 et SW480 ainsi que les cellules murines B16(F0) et RENCA (carcinome du rein, Murphy et al. J. Natl Cancer Inst., 1973. 50(4) : 1013-25) sont infectées par Ad null, Ad.CMV-FCU1 ou Ad.CMV-TK. Les cellules sont cultivées en présence de différentes concentrations de 5-FC ou de GCV. Après 10 jours de culture, la viabilité des cellules est déterminée par un comptage au bleu trypan. L'index thérapeutique (tableau 7) correspond au rapport LD50 des cellules non infectées (ou LD50 des cellules infectées par Ad null)/LD50 des cellules infectées par Ad.CMV-FCU1 ou par Ad.CMV-TK. De façon générale, l'index thérapeutique du système FCU1/5-FC est supérieure à celui du système TK/GCV, indiquant une meilleure efficacité thérapeutique de la stratégie gène suicide basée sur l'association FCU1/5-FC.

**Tableau 7**

| | **Index thérapeutique FCU1/5-FC** | **Index thérapeutique TK/GCV** |
|---|---|---|
| **B16(F0)** MOI 50 | 30 | 10 |
| **RENCA** MOI 5 | 100 | 100 |
| **SW480** MOI 1 | 300 | 100 |
| **PANC-1** MOI 1 | 1000 | 150 |
| **SK-BR-3** MOI 1 | 3000 | 1000 |

### 5. Expériences in vivo

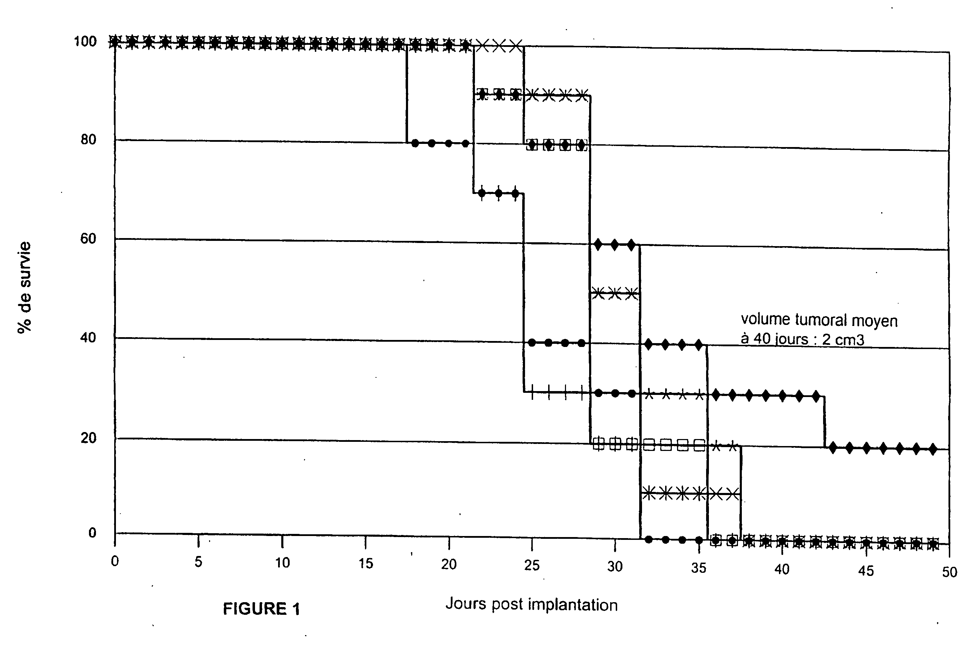

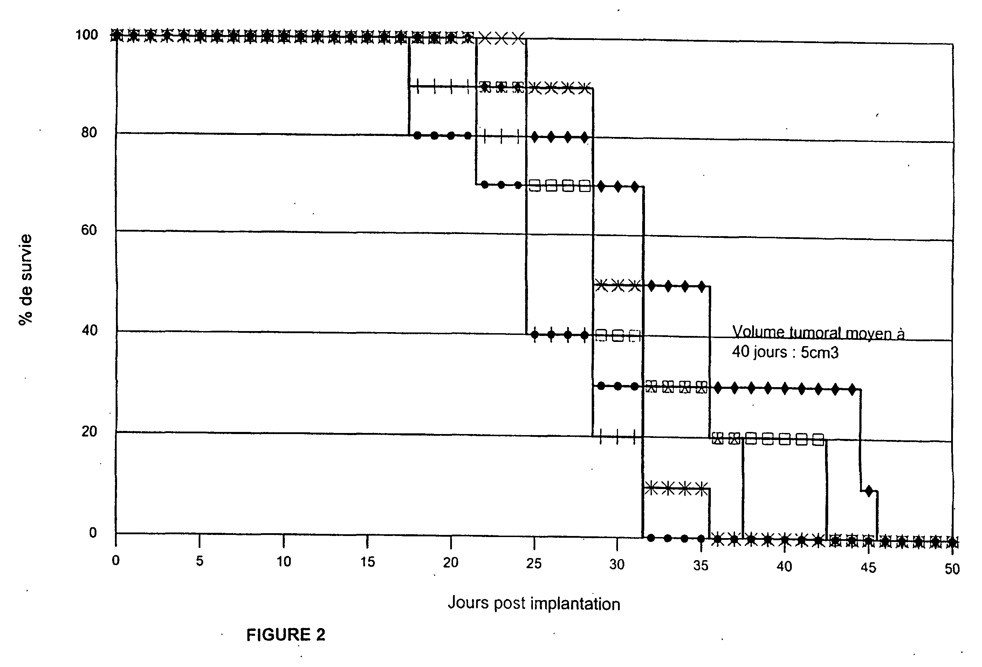

Afin d'évaluer la capacité des systèmes FCU1/5-FC et TK/GCV à inhiber la croissance des tumeurs *in vivo,* 3.10⁵ cellules B16(F0) sont injectées en sous cutané dans 6 groupes de n=10 souris immunocompétentes B6D2(=J0). Dès que les tumeurs deviennent palpables (J+8), un tampon « vehicle » (10 mM Tris pH8, 1 mM MgCl₂) ou les adénovirus (voir la légende des figures 1 et 2) resuspendus dans ce même tampon sont injectés par voie intratumorale à une dose de 5.10⁸ IU à trois reprises (J+8, J+9 et J+10). A partir de J+8, 1 ml d'une solution saline à 0,9%, ou 1 ml d'une solution de 5-FC à 1 % ou 1 ml d'une solution de GCV à 0,1 % est injectée par voie intrapéritonéale deux fois par jour jusqu'à J+43. Les résultats (Fig.1 et Fig.2) mettent en évidence une augmentation de survie dans le groupe injecté par l'Ad.CMV-FCU1 et traité au 5-FC par rapport au groupe Ad.CMV-TK traité au GCV et aux groupes contrôles. Ces résultats confirment l'intérêt de l'association du gène *FCU1* et de la prédrogue 5-FC notamment dans la mise en oeuvre d'un traitement antitumoral.

### EXEMPLE 7 Combinaison gène suicide FCU1 et gènes codant pour des cytokines.

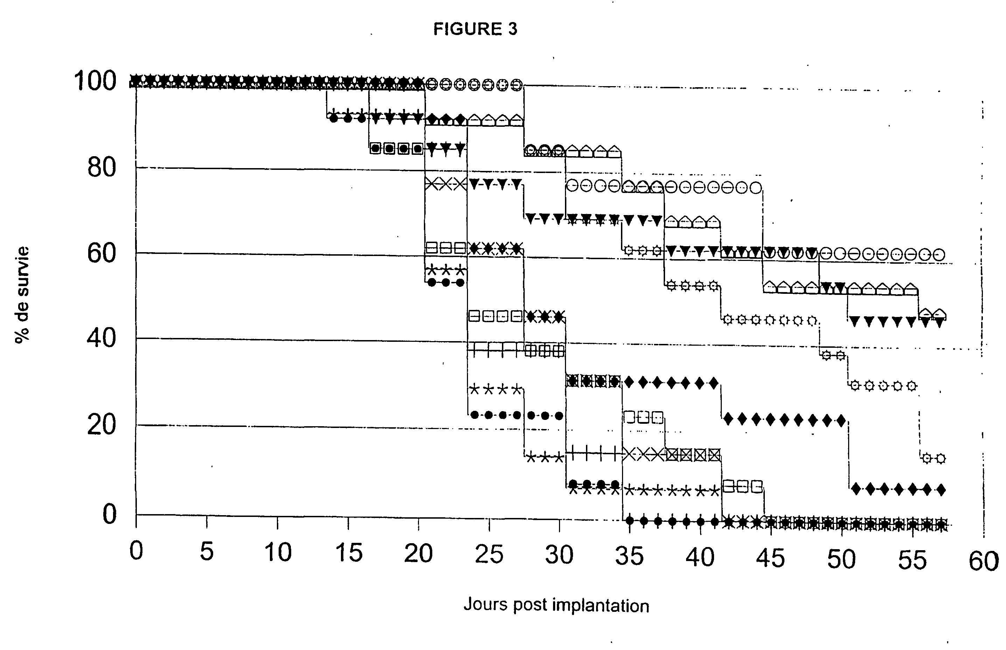

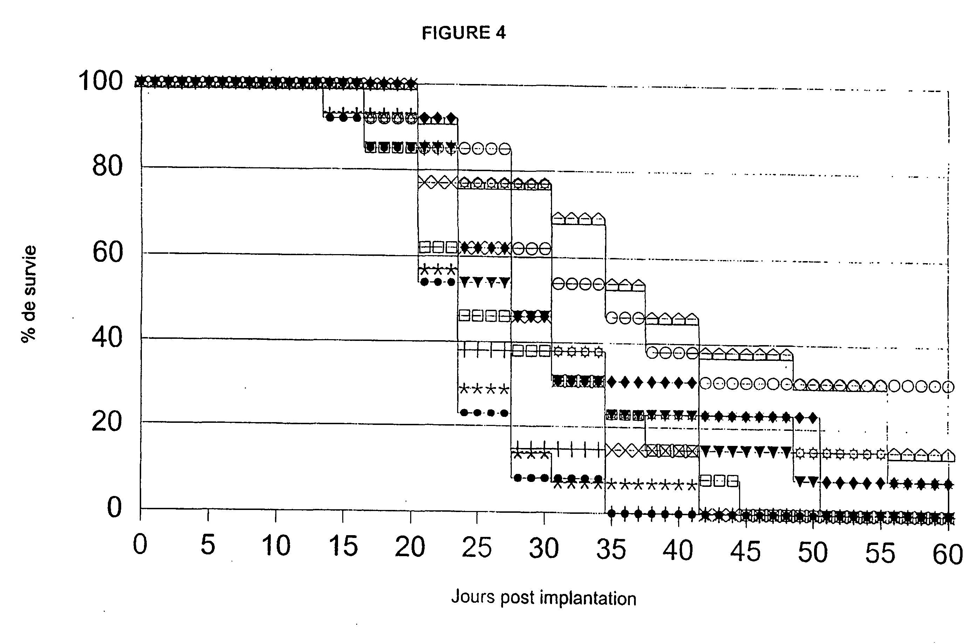

La combinaison FCU1 et cytokines (IFNγ et IL-2) est testée *in vivo* dans le modèle de tumeur murine B16(F0) implantée dans les souris immunocompétentes B6D2. Les adénovirus codant pour les cytokines sont l'Ad.CMV-muIFNγ (AdTG13048) exprimant l'IFNγ murin et l'Ad.CMV-huIL-2 (AdTG6624) exprimant l'IL-2 humain.
Le vecteur pTG13048 pour l'expression de l'IFNγ murin a été construit de la manière suivante : *le* fragment *Sal*I*-Not*I de pTG8390 renfermant le cDNA de l'IFNγ murin (muIFNγ) est isolé et introduit dans le vecteur pTG6600 linéarisé par *Xho*I*-Not*I, pour donner le vecteur de transfert pTG13047. La recombinaison homologue entre le fragment *Pac*I*-Bst*EII portant muIFNγ et isolé de pTG13047 *et le* vecteur pTG6624 linéarisé par *Cla*I permet de générer le vecteur adénoviral pTG13048 délété des régions E1 et E3 et comportant à la place de E1 le gène muIFNγ placé sous le contrôle du promoteur CMV et des séquences d'épissage hybride β-globine/Ig. Les particules virales (Ad.CMV-muIFNγ) sont obtenues par transfection de cellules 293. Le vecteur pTG6624 exprimant l'IL-2 humaine est décrit dans le document FR-A-2 763 959.
3.10⁵ cellules B16(F0) sont injectées en sous cutané dans les souris de 10 groupes de 13 souris immunocompétentes B6D2 (=J0). Dès que les tumeurs deviennent palpables (J+7), un tampon « vehicle » (10 mM Tris pH8, 1 mM MgCl₂) ou les adénovirus ou une combinaison d'adénovirus (voir la légende des figures 3 et 4) resuspendus dans ce même tampon sont injectés par voie intratumorale à une dose de 2.10⁸ IU par adénovirus (4.10⁸ IU au total) à trois reprises (J+7, J+8 et J+9). A partir de J+7, 1 ml d'une solution saline à 0,9% ou 1 ml d'une solution de 5-FC à 1% sont injectées par voie intrapéritonéale deux fois par jour. Les courbes de survie (Fig.3 et Fig.4) mettent en évidence une synergie entre le gène suicide et la cytokine entrant dans la composition de l'invention. Cette synergie est illustrée par un taux de survie supérieur dans le cas des combinaisons FCU1/cytokine par rapport à l'approche consistant à utiliser un adénovirus codant pour le gène suicide seul ou une cytokine seule.

## Revendications

1. Polypeptide ayant une activité uracile phosphoribosyl transférase (UPRTase), **caractérisé en ce qu'**il dérive d'une UPRTase native par délétion de la région N-terminale en amont du second codon ATG de ladite UPRTase native.

2. Polypeptide selon la revendication 1, **caractérisé par** une séquence en acides aminés telle que représentée à l'identificateur de séquence IDS NO: 1, débutant au résidu Met en position 1 et se terminant au résidu Val en position 216.

3. Polypeptide selon la revendication 2, **caractérisé en ce qu'**il comprend en outre une substitution du résidu sérine en position 2 par un résidu alanine.

4. Polypeptide selon l'unc des revendications 1 à 3, **caractérisé en ce qu'**il présente une activité UPRTase sensiblement plus élevée que celle présentée par ladite UPRTase native.

5. Polypeptide selon l'une des revendications 1 à 4, **caractérisé en ce qu'**il s'agit d'un polypeptide de fusion dans lequel ledit polypeptide est fusionné en phase avec au moins un second polypeptide.

6. Polypeptide selon la revendication 5, **caractérisé en ce qu'**il est fusionné en phase à un second polypeptide présentant une activité cytosine désaminase (CDase) et dérivant d'une cytosine désaminase native.

7. Polypeptide selon la revendication 5 ou 6, **caractérisé en ce que** la fusion avcc le second polypeptide est réalisée à l'extrémité N-terminale dudit polypeptide.

8. Polypeptide selon l'une des revendications 6 et 7, **caractérisé en ce que** ladite CDase native est de levure et en particulier celle codée par le gène *FCY1* de *Saccharomyces cerevisiae*.

9. Polypeptide selon la revendication 8, **caractérisé en ce qu'**il comprend la séquence en acides aminés représentée à l'identificateur de séquence IDS NO: 2, débutant au résidu Met en position 1 et se terminant au résidu Val en position 373.

10. Polypeptide selon l'une des revendications 5 à 9, **caractérisé en ce qu'**il présente une activité CDase plus élevée que ladite CDase native.

11. Séquence nucléutidique codant pour un polypeptide selon l'une des revendications 1 à 10.

12. Vecteur recombinant portant une séquence nucléotidique selon la revendication 11 placée sous le contrôle des éléments nécessaires à son expression dans une cellule hôte.

13. Vecteur recombinant selon la revendication 12, **caractérisé en ce que** ledit vecteur est choisi parmi le groupe constitué par les vecteurs plasmidiques et viraux éventuellement associé à une ou plusieurs substances améliorant l'eflicacité transfectionnelle et/ou la stabilité du vecteur.

14. Vecteur recombinant selon la revendication 13, **caractérisé en ce que** ledit vecteur est un vecteur viral dérivant d'un poxvirus, d'un adénovirus, d'un rétrovirus, d'un virus de l'herpès, d'un alphavirus, d'un foamyvirus ou d'un virus associé à l'adénvoirus.

15. Vecteur recombinant selon la revendication 13 ou 14, **caractérisé en ce que** ledit vecteur est un vecteur adénoviral dépourvu de tout ou partie d'au moins une région essentielle à la réplication sélectionnée parmi les régions E1, E2, E4 et L 1-L5.

16. Vecteur recombinant selon la revendication 15, **caractérisé en ce que** ledit vecteur est un vecteur adénoviral dépourvu en outre de tout ou partie de la région E3 non essentielle.

17. Vecteur recombinant selon l'une des revendications 12 à 16, **caractérisé en ce que** les éléments nécessaires à l'expression comprennent un promoteur et, en particulier le promoteur précoce du Cytomégalovirus (CMV).

18. Vecteur recombinant selon l'une des revendications 12 à 17, **caractérisé en ce qu'**il comprend en outre un ou plusieurs gènes d'intérêt choisi parmi les gènes codant pour les interleukines IL-2, IL-4, IL-7, IL-10, IL-12, les interférons, le facteur de nécrose des tumeurs (TNF), les facteurs stimulateurs des colonies (CSF), les facteurs agissant sur l'angiogénèse.

19. Vecteur recombinant selon la revendication 18, **caractérisé en ce que** le gène d'intérêt code pour un polypeptide choisi parmi l'IL-2 et l'INFγ.

20. Particule virale comprenant un vecteur recombinant selon l'une des revendications 14 à 19.

21. Cellule hôte comprenant une séquence nucléotidique selon la revendication 11, un vecteur recombinant selon l'une des revendications 12 à 19 ou infectée par une particule virale selon la revendication 20.

22. Composition comprenant un polypeptide selon l'une des revendications 1 à 10, une séquence nucléotidique selon la revendication 11, un vecteur recombinant selon l'une des revendications 12 à 19, une particule virale selon la revendication 20 ou une cellule hôte selon la revendication 21 en association avec un véhicule acceptable d'un point de vue pharmaceutique.

23. Composition selon la revendication 22, **caractérisée en ce qu'**elle comprend un polypeptide selon les revendications 1 à 4 et un second polypeptide d'intérêt, notamment un polypeptide présentant une activité CDase.

24. Composition selon la revendication 22, **caractérisée en ce qu'**elle comprend une séquence nucléotidique selon la revendication 11 codant pour un polypeptide selon l'une des revendications 1 à 4 et une seconde séquence nucléotidique d'intérêt, codant notamment pour un polypeptide présentant une activité CDase.

25. Composition selon la revendication 22, **caractérisée en ce qu'**elle comprend un polypeptide selon les revendications 1 à 10 et un second polypeptide d'intérêt, notamment un polypeptide choisi parmi l'IL-2 et l'INFγ.

26. Composition selon la revendication 22, **caractérisée en ce qu'**elle comprend une séquence nucléotidique selon la revendication 11 et une seconde séquence nucléotidique d'intérêt codant pour un polypeptide choisi parmi l'IL-2 et l'INFγ.

27. Utilisation thérapeutique ou prophylactique d'un polypeptide selon l'une des revendications 1 à 10, d'une séquence nucléotidique selon la revendication 11, d'un vecteur recombinant selon l'une des revendications 12 à 19, d'une particule virale selon la revendication 20 ou d'une cellule hôte selon la revendication 21 pour la préparation d'un médicament destiné au traitement des cancers, tumeur et maladies résultant d'une prolifération cellulaire non désirée.

28. Procédé de préparation d'un polypeptide selon l'une des revendications 1 à 10, selon lequel:
- on introduit une séquence nucléotidique selon la revendication 13 dans une cellule pour générer une cellule transformée
- on cultive ladite cellule transformée dans des conditions appropriées pour permettre la production dudit polypeptide, et
- on récolte ledit polypeptide de la culture cellulaire.

29. Vecteur recombinant selon la revendication 14, **caractérisé en ce que** ledit vecteur viral est un vecteur poxviral dérivant du virus de la vaccine.

30. Vecteur recombinant selon la revendication 14, **caractérisé en ce que** ledit vecteur viral est un vecteur poxviral dérivant du virus du MVA.

31. Procédé de préparation d'une particule virale, selon lequel :
(i) on introduit un vecteur recombinant selon l'une des revendications 14 à 16, 29 et 30, dans une cellule de complémentation capable de complémenter *en trans* ledit vecteur, de manière à obtenir une cellule de complémentation transfectée,
(ii) on cultive ladite cellule de complémentation transfectée dans des conditions appropriées pour permettre la production de ladite particule virale, et
(iii) on récupère ladite particule virale dans la culture cellulaire.

## Patentansprüche

1. Polypeptid mit Uracil-Phosphoribosyl-Tranaferase-Aktivität (UPRTase-Aktivität), **dadurch gekennzeichnet, daß** es sich von einer nativen UPRTase durch Deletion der N-terminalen Region stromaufwärts des zweiten ATG-Codons dieser nativen UPRTase ableitet.

2. Polypeptid nach Anspruch 1, das durch eine Aminosäuresequenz wie in der Bezeichnung der Sequenz ID NO: 1 angegeben ist, **gekennzeichnet** ist, wobei der Anfang bei dem Met-Rest in Position 1 und das Ende am Val-Rest in Position 216 liegt.

3. Polypeptid nach Anspruch 2, **dadurch gekennzeichnet, daß** es weiterhin eine Substitution des Serinrests in Position 2 durch einen Alaninrest umfaßt.

4. Polypeptid nach einem der Ansprüche 1 bis 3,
**dadurch gekennzeichnet, daß** es eine wesentlich höhere UPRTase-Aktivität als die native UPRTase aufweist.

5. Polypeptid nach einem der Ansprüche 1 bis 4, **dadurch gekennzeichnet, daß** es sich um ein Fusionspolypeptid handelt, in dem das Polypeptid leserastergerecht mit mindestens einem zweiten polypeptid fusioniert ist.

6. Polypeptid nach Anspruch 5, **dadurch gekennzeichnet, daß** es leserastergerecht mit einem zweiten Polypeptid, das eine Cytosindesaminase-Aktivität (CDase-Aktivität) aufweist und sich von einer nativen Cytosindesaminase ableitet, fusioniert ist.

7. Polypeptid nach Anspruch 5 oder 6, **dadurch gekennzeichnet, daß** die Fusion mit dem zweiten Polypeptid am N-terminalen Ende dieses Polypeptids stattfindet.

8. Polypeptid nach Anspruch 6 oder 7, **dadurch gekennzeichnet, daß** die native CDase von Hefe stammt und insbesondere die von dem FCYI-Gen von *Saccharomyces cerevisiae* codierte CDase ist.

9. Polypeptid nach Anspruch 8, das durch eine Aminosäuresequenz wie in der Bezeichnung der Sequenz ID NO: 2 angegeben ist, **gekennzeichnet** ist, wobei der Anfang bei dem Met-Rest in Position 1 und das Ende am Val-Rest in Position 373 liegt.

10. Polypeptid nach einem der Ansprüche 5 bis 9, **dadurch gekennzeichnet, daß** es eine höhere CDase-Aktivität als die nativen CDase aufweist.

11. Nukleotidsequenz, die für ein Polypeptid nach einem der Ansprüche 1 bis 10 codiert.

12. Rekombinanter Vektor, der eine Nukleotidsequenz nach Anspruch 11 unter der Kontrolle von für seine Expression in einer Wirtszelle erforderlichen Elementen trägt.

13. Rekombinanter Vektor nach Anspruch 12, **dadurch gekennzeichnet, daß** der Vektor aus der Gruppe der Plasmid- und Virusvektoren, gegebenenfalls in Kombination mit einer oder mehreren Substanzen, die die Transfektionseffizienz und/oder die Stabilität des Vektors verbessern, stammt.

14. Rekombinanter Vektor nach Anspruch 13, **dadurch gekennzeichnet, daß** es sich bei dem Vektor um einen Virusvektor handelt, der sich von einem Poxvirus, von einem Adenovirus, von einem Retrovirus, von einem Herpesvirus, von einem Alphavirus, von einem Foamyvirus oder von einem adenovirus-assoziierten Virus ableitet.

15. Rekombinanter Vektor nach Anspruch 13 oder 14, **dadurch gekennzeichnet, daß** es sich bei dem Vektor um einen Adenovirusvektor handelt, dem mindestens eine für seine Replikation erforderliche Region aus der Gruppe der Regionen E1, E2, E4 und L1-L5 ganz oder teilweise fehlt.

16. Rekombinanter Vektor nach Anspruch 15, **dadurch gekennzeichnet, daß** es sich bei dem Vektor um einen Adenovirusvektor handelt, dem weiterhin die nichtessentielle Region E3 ganz oder teilweise fehlt.

17. Rekombinanter Vektor nach einem der Ansprüche 12 bis 16, **dadurch gekennzeichnet, daß** die für die Expression erforderlichen Elemente einen Promoter, insbesondere den frühen Cytomegalivirus (CMV)-Promoter, umfaßt.

18. Rekombinanter Vektor nach einem der Ansprüche 12 bis 17, **dadurch gekennzeichnet, daß** er weiterhin ein oder mehrere interessierende Gene aus der Reihe der Gene, die für die Interleukine IL-2, IL-4, IL-7, IL-10, IL-12, die Interferone, den Tumornekrosefaktor (TNF), die Kolonie stimulierenden Faktoren (CSF) und Faktoren, die die Angiogenese beeinflussen, umfaßt.

19. Rekombinanter Vektor nach Anspruch 18, **dadurch gekennzeichnet, daß** das interessierende Gen für ein Polypeptid aus der Reihe IL-2 und INFγ codiert.

20. viruspartikel, das einen rekombinanten Vektor nach einem der Ansprüche 14 bis 19 umfaßt.

21. Wirtszelle, die eine Nukleotidsequenz nach Anspruch 11, einen rekombinanten Vektor nach einem der Ansprüche 12 bis 19 umfaßt oder mit einem Viruspartikel nach Anspruch 20 infiziert ist.

22. Zusammensetzung, die ein Polypeptid nach einem der Ansprüche 1 bis 10, eine Nukleotidsequenz nach Anspruch 11, einen rekombinanten Vektor nach einem der Ansprüche 12 bis 19, ein Viruspartikel nach Anspruch 20 oder eine Wirtszelle nach Anspruch 21 in Kombination mit einem pharmazeutisch annehmbaren Grundstoff umfaßt.

23. Zusammensetzung nach Anspruch 22, **dadurch gekennzeichnet, daß** sie ein Polypeptid nach den Ansprüchen 1 bis 4 sowie ein zweites interessierendes Polypeptid, insbesondere ein Polypeptid mit CDase-Aktivität, umfaßt.

24. Zusammensetzung nach Anspruch 22, **dadurch gekennzeichnet, daß** sie eine Nukleotidsequenz nach Anspruch 11, die für ein Polypeptid nach einem der Ansprüche 1 bis 4 codiert, sowie eine zweite interessierende Nukleotidsequenz, die insbesondere für ein Polypeptid mit CDase-Aktivität codiert, umfaßt.

25. Zusammensetzung nach Anspruch 22, **dadurch gekennzeichnet, daß** sie ein Polypeptid nach den Ansprüchen 1 bis 10 sowie ein zweites interessierendes Polypeptid, insbesondere ein Polypeptid aus der Reihe IL-2 und INFγ, umfaßt.

26. Zusammensetzung nach Anspruch 22, **dadurch gekennzeichnet**, das sie eine Nukleotidsequenz nach Anspruch 11 sowie eine zweite interessierende Nukleotidsequenz, die für ein Polypeptid aus der Reihe IL-2 und INFγ codiert, umfaßt.

27. Therapeutische oder prophylaktische Verwendung eines Polypeptids nach einem der Ansprüche 1 bis 10, einer Nukleotidsequenz nach Anspruch 11, eines rekombinanten Vektors nach einem der Ansprüche 12 bis 19, eines Viruspartikels nach Anspruch 20 oder einer Wirtszelle nach Anspruch 21 zur Herstellung eines Arzneimittels für die Behandlung von Krebs, Tumor und Krankheiten, die durch eine unerwünschte Zellproliferation entstehen.

28. Verfahren zur Herstellung eines Polypeptids nach einem der Ansprüche 1 bis 10, bei dem man
- eine Nukleotidsequenz nach Anspruch 13 in eine Zelle einbringt, wodurch man eine transformierte Zelle erzeugt,
- diese transformierte Zelle unter geeigneten Bedingungen, die die Produktion des Polypeptids gestatten, züchtet, und
- das Polypeptid aus der Zellkultur erntet.

29. Rekombinanter Vektor nach Anspruch 14, **dadurch gekennzeichnet, daß** es sich bei dem Virusvektor um einen Poxvirusvektor, der sich von einem Vaccinevirus ableitet, handelt.

30. Rekombinanter Vektor nach Anspruch 14, **dadurch gekennzeichnet, daß** es sich bei dem Virusvektor um einen Poxviruavektor, der sich von einem MVA-Virus ableitet, handelt.

31. Verfahren zur Herstellung eines Viruspartikels, bei dem man:
(i) einen rekombinanten Vektor nach einem der Ansprüche 14 bis 16, 29 und 30 in eine Komplementierungszelle, die diesen Vektor zu *trans*-komplementieren vermag, um zu einer transfizierten Komplementierungszelle zu gelangen,
(ii) man züchtet diese transfizierte Komplementierungszelle unter geeigneten Bedingungen, die die Produktion des Viruspartikels gestatten und
(iii) man gewinnt das Viruspartikel in der Zellkultur.

## Claims

1. Polypeptide possessing a uracil phosphoribosyl transferase (UPRTase) activity, **characterized in that** it is derived from a native UPRTase by deleting the N-terminal region upstream of the second ATG codon of said native UPRTase.

2. Polypeptide according to Claim 1, **characterized by** an amino acid sequence as depicted in the SEQ ID: NO:1 sequence identifier, starting at the Met residue in position 1 and finishing at the Val residue in position 216.

3. Polypeptide according to Claim 2, **characterized in that** it additionally comprises a replacement of the serine residue in position 2 with an alanine residue.

4. Polypeptide according to one of Claims 1 to 3, **characterized in that** it exhibits a UPRTase activity which is appreciably higher than that exhibited by said native UPRTase.

5. Polypeptide according to one of Claims 1 to 4, **characterized in that** it is a fusion polypeptide in which said polypeptide is fused in-frame with at least a second polypeptide.

6. Polypeptide according to Claim 5, **characterized in that** it is fused in-frame with a second polypeptide which exhibits a cytosine deaminase (CDase) activity and is derived from a native cytosine deaminase.

7. Polypeptide according to Claim 5 or 6, **characterized in that** the fusion with the second polypeptide is carried out at the N-terminal end of said polypeptide.

8. Polypeptide according to either of Claims 6 and 7, **characterized in that** said native CDase is a yeast CDase, in particular that encoded by the *Saccharomyces cerevisiae FCY1* gene.

9. Polypeptide according to Claim 8, **characterized in that** it comprises the amino acid sequence depicted in the SEQ ID NO: 2 sequence identifier, starting at the Met residue in position 1 and finishing at the Val residue in position 373.

10. Polypeptide according to one of Claims 5 to 9, **characterized in that** it exhibits a higher CDase activity than said native CDase.

11. Nucleotide sequence which encodes a polypeptide according to one of Claims 1 to 10.

12. Recombinant vector which carries a nucleotide sequence according to Claim 11 placed under the control of the elements which are required for expressing it in a host cell.

13. Recombinant vector according to Claim 12, **characterized in that** said vector is selected from the group consisting of the plasmid and viral vectors, where appropriate combined with one or more substances which improve(s) the transfectional efficacy and/or the stability of the vector.

14. Recombinant vector according to Claim 13, **characterized in that** said vector is a viral vector which is derived from a poxvirus, from an adenovirus, from a retrovirus, from a herpesvirus, from an alphavirus, from a foamyvirus or from an adenovirus-associated virus.

15. Vector according to Claim 13 or 14, **characterized in that** said vector is an adenoviral vector which lacks all or part of at least one region which is essential for replication and which is selected from the E1, E2, E4 and L1-L5 regions.

16. Vector according to Claim 15, **characterized in that** said vector is an adenoviral vector which additionally lacks all or part of the non-essential E3 region.

17. Recombinant vector according to one of Claims 12 to 16, **characterized in that** the elements which are required for the expression comprise a promoter, in particular the cytomegalovirus (CMV) early promoter.

18. Recombinant vector according to one of Claims 12 to 17, **characterized in that** it additionally comprises one or more genes of interest which is/are selected from the genes encoding interleukins IL-2, IL-4, IL-7, IL-10 and IL-12, interferons, tumor necrosis factor (TNF), colony stimulating factors (CSF) and factors acting on angiogenesis.

19. Recombinant vector according to Claim 18, **characterized in that** the gene of interest encodes a polypeptide which is selected from IL-2 and INFγ.

20. Viral particle which comprises a recombinant vector according to one of Claims 14 to 19.

21. Host cell which comprises a nucleotide sequence according to Claim 11 or a recombinant vector according to one of Claims 12 to 19, or which is infected with a viral particle according to Claim 20.

22. Composition which comprises a polypeptide according to one of Claims 1 to 10, a nucleotide sequence according to Claim 11, a recombinant vector according to one of Claims 12 to 19, a viral particle according to Claim 20 or a host cell according to Claim 21, in combination with a pharmaceutically acceptable excipient.

23. Composition according to Claim 22, **characterized in that** it comprises a polypeptide according to Claims 1 to 4 and a second polypeptide of interest, in particular a polypeptide which exhibits a CDase activity.

24. Composition according to Claim 22, **characterized in that** it comprises a nucleotide sequence according to Claim 11 which encodes a polypeptide according to one of Claims 1 to 4, and a second nucleotide sequence of interest which encodes, in particular, a polypeptide which exhibits a CDase activity.

25. Composition according to Claim 22, **characterized in that** it comprises a polypeptide according to Claims 1 to 10 and a second polypeptide of interest, in particular a polypeptide selected from IL-2 and INFγ.

26. Composition according to Claim 22, **characterized in that** it comprises a nucleotide sequence according to Claim 11 and a second nucleotide sequence of interest which encodes a polypeptide selected from IL-2 and INFγ.

27. Therapeutic or prophylactic use of a polypeptide according to one of Claims 1 to 10, of a nucleotide sequence according to Claim 11, of a recombinant vector according to one of Claims 12 to 19, of a viral particle according to Claim 20 or of a host cell according to Claim 21 for preparing a medicament which is intended for treating cancers, tumors and diseases which result from unwanted cell proliferation.

28. Process for preparing a polypeptide according to one of Claims 1 to 10, in which:
- a nucleotide sequence according to Claim 13 is introduced into a cell in order to generate a transformed cell
- said transformed cell is cultured under conditions which are appropriate for enabling said polypeptide to be produced, and
- said polypeptide is harvested from the cell culture.

29. Recombinant vector according to Claim 14 **characterized in that** the said viral vector is a poxviral vector which is derived from the vaccinia virus.

30. Recombinant vector according to Claim 14, **characterized in that** the said viral vector is a poxviral vector which is derived from the MVA virus.

31. Process for preparing a viral particle according to which:
(i) a recombinant vector according to one of Claims 14 to 16, 29 and 30 is introduced into a complementation cell which is able to complement the said vector in trans so as to obtain a transfected complementation cell.
(ii) the said transfected complementation cell is cultured under conditions which are appropriate for producing the said viral particle, and
(iii) the said viral particle is recovered from the cell culture.


### LISTE DE SEQUENCES

(1) INFORMATION GENERALE:
   (i) DEPOSANT:
      (A) NOM: TRANSGENE S.A.
      (B) RUE: 11 rue de Molsheim
      (C) VILLE: Strasbourg
      (E) PAYS: France
      (F) CODE POSTAL: 67000
      (G) TELEPHONE: (0)3 88 27 91 00
      (H) TELECOPIE: (0)3 88 27 91 41
   (ii) TITRE DE L' INVENTION: Mutant ayant une activite uracile phosphoribosyl transferase.
   (iii) NOMBRE DE SEQUENCES: 8
   (iv) FORME LISIBLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 216 acides aminés
      (B) TYPE: acide aminé
      (D) CONFIGURATION: linéaire
   (ii) TYPE DE MOLECULE: protéine
   (iii) HYPOTHETIQUE: NON
   (vi) ORIGINE:
      (A) ORGANISME: Saccharomyces cerevisiae
   (vii) SOURCE IMMEDIATE:
      (B) CLONE: mutant UPRTase delta 35
   (xi) DESCRIPTION DE LA SEQUENCE: SEQ ID NO: 1:
   Met Ala Ser Glu Pro Phe Lys Asn Val Tyr Leu Leu Pro Gln Thr Asn
   1               5                   10                  15
   Gln Leu Leu Gly Leu Tyr Thr Ile Ile Arg Asn Lys Asn Thr Thr Arg
               20                  25                  30
   Pro Asp Phe Ile Phe Tyr Ser Asp Arg Ile Ile Arg Leu Leu Val Glu
           35                  40                  45
   Glu Gly Leu Asn His Leu Pro Val Gln Lys Gln Ile Val Glu Thr Asp
       50                  55                  60
   Thr Asn Glu Asn Phe Glu Gly Val Ser Phe Met Gly Lys Ile Cys Gly
   65                  70                  75                  80
   Val Ser Ile Val Arg Ala Gly Glu Ser Met Glu Gln Gly Leu Arg Asp
                   85                  90                  95
   Cys Cys Arg Ser Val Arg Ile Gly Lys Ile Leu Ile Gln Arg Asp Glu
               100                 105                 110
   Glu Thr Ala Leu Pro Lys Leu Phe Tyr Glu Lys Leu Pro Glu Asp Ile
           115                 120                 125
   Ser Glu Arg Tyr Val Phe Leu Leu Asp Pro Met Leu Ala Thr Gly Gly
       130                 135                 140
   Ser Ala Ile Met Ala Thr Glu Val Leu Ile Lys Arg Gly Val Lys Pro
   145                 150                 155                 160
   Glu Arg Ile Tyr Phe Leu Asn Leu Ile Cys Ser Lys Glu Gly Ile Glu
                   165                 170                 175
   Lys Tyr His Ala Ala Phe Pro Glu Val Arg Ile Val Thr Gly Ala Leu
               180                 185                 190
   Asp Arg Gly Leu Asp Glu Asn Lys Tyr Leu Val Pro Gly Leu Gly Asp
           195                 200                 205
   Phe Gly Asp Arg Tyr Tyr Cys Val
       210                 215
(2) INFORMATION POUR LA SEQ ID NO: 2:
   (i) CARACTERISTIQUES DE LA SEQUENCE:
      (A) LONGUEUR: 373 acides aminés
      (B) TYPE: acide aminé
      (D) CONFIGURATION: linéaire
   (ii) TYPE DE MOLECULE: protéine
   (iii) HYPOTHETIQUE: NON
   (iii) ANTI-SENS: NON
   (vi) ORIGINE:
      (A) ORGANISME: Saccharomyces cerevisiae
   (vii) SOURCE IMMEDIATE:
      (B) CLONE: proteine de fusion fcy1::fur1 delta 35 (FCU1)
   (xi) DESCRIPTION DE LA SEQUENCE: SEQ ID NO: 2:
   Met Val Thr Gly Gly Met Ala Ser Lys Trp Asp Gln Lys Gly Met Asp
   1               5                   10                  15
   Ile Ala Tyr Glu Glu Ala Ala Leu Gly Tyr Lys Glu Gly Gly Val Pro
               20                  25                  30
   Ile Gly Gly Cys Leu Ile Asn Asn Lys Asp Gly Ser Val Leu Gly Arg
               35                          40                          45
   Gly His Asn Met Arg Phe Gln Lys Gly Ser Ala Thr Leu His Gly Glu
       50                  55                      60
   Ile Ser Thr Leu Glu Asn Cys Gly Arg Leu Glu Gly Lys Val Tyr Lys
   65                  70                  75                      80
   Asp Thr Thr Leu Tyr Thr Thr Leu Ser Pro Cys Asp Met Cys Thr Gly
                   85                  90                      95
   Ala Ile Ile Met Tyr Gly Ile Pro Arg Cys Val Val Gly Glu Asn Val
               100                 105                     110
   Asn Phe Lys Ser Lys Gly Glu Lys Tyr Leu Gln Thr Arg Gly His Glu
           115                 120                 125
   Val Val Val Val Asp Asp Glu Arg Cys Lys Lys Ile Met Lys Gln Phe
       130                 135                 140
   Ile Asp Glu Arg Pro Gln Asp Trp Phe Glu Asp Ile Gly Glu Ala Ser
   145                 150                 155                     160
   Glu Pro Phe Lys Asn Val Tyr Leu Leu Pro Gln Thr Asn Gln Leu Leu
                   165                 170                     175
   Gly Leu Tyr Thr Ile Ile Arg Asn Lys Asn Thr Thr Arg Pro Asp Phe
               180                 185                     190
   Ile Phe Tyr Ser Asp Arg Ile Ile Arg Leu Leu Val Glu Glu Gly Leu
           195                 200                 205
   Asn His Leu Pro Val Gln Lys Gln Ile Val Glu Thr Asp Thr Asn Glu
       210                 215                 220
   Asn Phe Glu Gly Val Ser Phe Met Gly Lys Ile Cys Gly Val Ser Ile
   225                 230                 235                     240
   Val Arg Ala Gly Glu Ser Met Glu Gln Gly Leu Arg Asp Cys Cys Arg
                   245                 250                     255
   Ser Val Arg Ile Gly Lys Ile Leu Ile Gln Arg Asp Glu Glu Thr Ala
               260                 265                 270
   Leu Pro Lys Leu Phe Tyr Glu Lys Leu Pro Glu Asp Ile Ser Glu Arg
           275                 280                 285
   Tyr Val Phe Leu Leu Asp Pro Met Leu Ala Thr Gly Gly Ser Ala Ile
       290                 295                 300
   Met Ala Thr Glu Val Leu Ile Lys Arg Gly Val Lys Pro Glu Arg Ile
   305                 310                 315                     320
   Tyr Phe Leu Asn Leu Ile Cys Ser Lys Glu Gly Ile Glu Lys Tyr His
                   325                 330                     335
   Ala Ala Phe Pro Glu Val Arg Ile Val Thr Gly Ala Leu Asp Arg Gly
               340                 345                     350
   Leu Asp Glu Asn Lys Tyr Leu Val Pro Gly Leu Gly Asp Phe Gly Asp
           355                 360                 365
   Arg Tyr Tyr Cys Val
       370
(2) INFORMATION POUR LA SEQ ID NO: 3:
   (i) CARACTERISTIQUES DE LA SEQUENCE:
      (A) LONGUEUR: 25 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Saccharomyces cerevisiae
   (vii) SOURCE IMMEDIATE:
      (B) CLONE: oligonucleotide de synthese
   (xi) DESCRIPTION DE LA SEQUENCE: SEQ ID NO: 3:
   CGGGTCCATG GTTGAATTCG AAATG                                        25
(2) INFORMATION POUR LA SEQ ID NO: 4:
   (i) CARACTERISTIQUES DE LA SEQUENCE:
      (A) LONGUEUR: 19 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Saccharomyces cerevisiae
   (vii) SOURCE IMMEDIATE:
      (B) CLONE: oligonucleotide de synthese
   (xi) DESCRIPTION DE LA SEQUENCE: SEQ ID NO: 4:
   TGGTGTACGC GTGTGATTT 19
(2) INFORMATION POUR LA SEQ ID NO: 5:
   (i) CARACTERISTIQUES DE LA SEQUENCE:
      (A) LONGUEUR: 26 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SEP1S: OUI
   (vi) ORIGINE:
      (A) ORGANISME: Saccharomyces cerevisiae
   (vii) SOURCE IMMEDIATE:
      (B) CLONE: oligonucleotide de synthese
   (xi) DESCRIPTION DE LA SEQUENCE: SEQ ID NO: 5:
   CGAAGCCATG GTTTGCAGAA TTCTAG 26
(2) INFORMATION POUR LA SEQ ID NO: 6:
   (i) CARACTERISTIQUES DE LA SEQUENCE:
      (A) LONGUEUR: 25 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Saccharomyces cerevisiae
   (vii) SOURCE IMMEDIATE:
      (B) CLONE: oligonucleotide de synthese
   (xi) DESCRIPTION DE LA SEQUENCE: SEQ ID NO: 6:
   GTTAAAAGCT TCATAGGCCA TGGTG 25
(2) INFORMATION POUR LA SEQ IP NO: 7:
   (i) CARACTERISTIQUES DE LA SEQUENCE:
      (A) LONGUEUR: 21 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SEPIS: NON
   (vi) ORIGINE:
      (A) ORGANISME: Saccharomyces cerevisiae
   (vii) SOURCE IMMEDIATE:
      (B) CLONE: oligonucleotide de synthese
   (xi) DESCRIPTION DE LA SEQUENCE: SEQ ID NO: 7:
(2) INFORMATION POUR LA SEQ ID NO: 8:
   (i) CARACTERISTIQUES DE LA SEQUENCE:
      (A) LONGUEUR: 26 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Saccharomyces cerevisiae
   (vii) SOURCE IMMEDIATE:
      (B) CLONE: oligonucleotide de synthese
   (xi) DESCRIPTION DE LA SEQUENCE: SEQ ID NO: 8:
   ATGGTTCCGA AGCCTCACCA ATATCT 26